# EUROPEAN PATENT APPLICATION

(11) **EP 4 349 365 A1**
(43) Date of publication of application: **10.04.2024**
(21) Application number: 22200431.9
(22) Date of filing: 07.10.2022
(51) Int. Cl.: A61K 38/48, A61K 38/47, A61K 48/00, C12N 15/861, A61P 1/00, A61P 3/00, A61P 5/00, A61P 7/00, A61P 9/00, A61P 11/00, A61P 21/00, A61P 25/00, A61P 31/00

(54) **CO-TREATMENT FOR GENE THERAPY**

(71) Applicant: Hansa Biopharma AB, 220 07 Lund (SE)
(72) Inventor: ERLANDSSON, Eva, SE-220 07 Lund (SE); UZCÁTEGUI, Nathalie, SE-220 07 Lund (SE); NILSSON, Sara, SE-220 07 Lund (SE); FREIBURGHAUS, Catja, SE-220 07 Lund (SE); LINDVALL, Mikaela, SE-220 07 Lund (SE); WINSTEDT, Lena, SE-220 07 Lund (SE); JAIN, Mayur, SE-220 07 Lund (SE); NILSSON, Helena Jernmark, SE-220 07 L:und (SE); ZHANG, Xingmei, SE-220 07 Lund (SE); KJELLMAN, Christian, SE-220 07 Lund (SE)
(74) Representative: J A Kemp LLP

(57) **Abstract**

The present invention relates to a method of enhancing gene therapy by using an enzyme effective to degrade or digest and/or inhibit or reduce effector function of serum antibodies in the subject. More particularly, the subject is one that has no detectable antibodies that specifically bind to the gene therapy, or has either a low titre of neutralising antibodies that specifically bind to the gene therapy (NAb) or a low titre of total antibodies that specifically bind to the gene therapy (TAb). The method may increase the serum half-life of the therapy and mean that a lower dose of the gene therapy can be administered than would otherwise be given.

## Description

### Field of the Invention

The present invention relates to gene therapy, including compositions and methods for improving gene therapy, particularly in terms of reducing the dose of the gene therapy agent needed and/or prolonging the effect of the gene therapy.

### Background of the Invention

Gene therapy is a fast-developing and hugely promising approach. One issue with gene therapy encountered though is that the body often generates an immune response against a gene therapy agent and that may be particularly the case after repeated doses of the gene therapy agent have been administered. Some gene therapy vectors are based on viruses which occur naturally, such as Adeno-Associated Virus (AAV), where infections with wild-type AAVs are common, representing a reason why a subject may already have antibodies specific for the gene therapy agent, prior to administration of the gene therapy.

Antibodies against therapeutic agents, particularly gene therapy agents, are known as Anti-Drug Antibodies (ADA). The generation of ADA can reduce the transduction efficiency of a gene therapy, as well as promoting more rapid clearance of the gene therapy agent. ADA can therefore reduce the efficiency of a gene therapy agent. Some individuals may have such a high titer of pre-existing ADA that administration of a gene therapy agent as a treatment is not considered appropriate for them at all. In other individuals such a high titer of ADA has developed after initial treatment with a gene therapy that it is no longer considered safe to given them further doses of the gene therapy, meaning that a treatment that was initially effective is no longer appropriate.

It may be that ADA, whether pre-existing or which has developed after initial gene therapy treatment, represents a barrier to a gene therapy being effective at all in a given individual. It may also be though that ADA means that such a high titer of gene therapy vector would need to be given to get the necessary effect safety concerns exist. Given the history of gene therapy, where adverse events have sometimes occurred, the safety of any gene therapy is a key factor and so anything that reduces the dosage of a gene therapy needed to be given is clearly desirable.

Given the impact of ADA there has therefore been a continued focus on trying to find ways to reduce the impact of ADA in gene therapy.

### Summary of the Invention

Unexpectedly, it has been found that even where a subject has low or no serum antibodies against a gene therapy agent it may still be beneficial to administer an enzyme that is capable of targeting serum antibodies prior to administration of a gene therapy. Given that ADA is commonly thought to be the cause of reduced efficiency for gene therapy, the finding that even with no, or low, levels of antibodies specific for a gene therapy agent there still appears a benefit in administering an enzyme that can target serum antibodies as well as the gene therapy agent is unexpected given the focus on ADA in the field.

It has also been unexpectedly found that for subjects in general the enzyme may be used to delay clearance of a gene therapy, in particular from the circulation, and hence increase the exposure of a given subject to a gene therapy. The invention may therefore also provide a way to reduce the dose of a gene therapy agent needing to be given to a subject owing to the increased exposure of the subject to the gene therapy agent.

Thus, the invention provides a way to enhance gene therapy in general and also to help treat specific patients for whom it would have been previously considered unnecessary to administer the enzyme to when giving the gene therapy. It appears that the cleavage products of the enzyme may have an unexpected inhibitory effect on the clearance of gene therapy vector. By slowing gene therapy vector clearance and keeping the vector in circulation the chance of reaching target organs can be improved.

A benefit of the invention may be therefore in terms of reducing the amount of gene therapy agent administered, for example to a lower dose than would otherwise be usually administered. A benefit may be, or additionally be, increasing the half-life of the gene therapy agent in the body once administered. A benefit may be that a bigger effect is seen per given amount of gene therapy agent. A benefit may be increasing the exposure of the subject to the gene therapy. A benefit may be in delaying the clearance time of a gene therapy. The benefit may be in terms of increasing the amount of the gene therapy agent reaching a target organ. The benefit may be in terms of be able to treat patient groups for which it was previously considered unnecessary to administer such enzymes. The benefit may be it terms of an increase in the rate of gene transduction. Any of those benefits may be seen individually or in combination with each other.

Accordingly, the present invention provides a method of treating a subject in need of gene therapy for a disease comprising:
a. administering to said subject an amount of an enzyme effective to degrade or digest and/or inhibit or reduce effector function of serum antibodies in the subject; and
b. administering the gene therapy to said subject;

wherein the reaction products resulting from the activity of the enzyme prolong the serum half-life of the gene therapy in the subject and/or prolong or increase the exposure of the subject to the gene therapy;
wherein the gene therapy is administered at a dose which is lower than the minimally effective dose that would be administered to a subject in the absence of step a.; and
wherein said subject has no detectable antibodies that specifically bind to the gene therapy, or has either a low titre of neutralising antibodies that specifically bind to the gene therapy (NAb) or a low titre of total antibodies that specifically bind to the gene therapy (TAb), optionally wherein the presence/titre of antibodies in the subject is measured in a serum sample obtained from the subject prior to step a.

The present invention further provides a gene therapy agent for use in a method of treating a subject in need of gene therapy for a disease comprising:
a. administering to said subject an amount of an enzyme effective to degrade or digest and/or inhibit or reduce effector function of serum antibodies in the subject; and
b. administering the gene therapy to said subject;

wherein the reaction products resulting from the activity of the enzyme prolong the serum half-life of the gene therapy in the subject and/or prolong or increase the exposure of the subject to the gene therapy;
wherein the gene therapy is administered at a dose which is lower than the minimally effective dose that would be administered to a subject in the absence of step a.; and
wherein said subject has no detectable antibodies that specifically bind to the gene therapy, or has either a low titre of neutralising antibodies that specifically bind to the gene therapy (NAb) or a low titre of total antibodies that specifically bind to the gene therapy (TAb), optionally wherein the presence/titre of antibodies in the subject is measured in a serum sample obtained from the subject prior to step a.

The present invention further provides an enzyme for use in a method of treating a subject in need of gene therapy for a disease comprising:
a. administering to said subject an amount of the enzyme wherein the enzyme is an enzyme effective to degrade or digest and/or inhibit or reduce effector function of serum antibodies in the subject; and
b. administering the gene therapy to said subject;

wherein the reaction products resulting from the activity of the enzyme prolong the serum half-life of the gene therapy in the subject and/or prolong or increase the exposure of the subject to the gene therapy;
wherein the gene therapy is administered at a dose which is lower than the minimally effective dose that would be administered to a subject in the absence of step a.; and
wherein said subject has no detectable antibodies that specifically bind to the gene therapy, or has either a low titre of neutralising antibodies that specifically bind to the gene therapy (NAb) or a low titre of total antibodies that specifically bind to the gene therapy (TAb), optionally wherein the presence/titre of antibodies in the subject is measured in a serum sample obtained from the subject prior to step a.

### Brief Description of the Figures

**Fig 1****. qPCR detection of AAV in SCID Mice plasma up to 7 days after AAV injection.** SCID mice were administered either: (a) the AAV gene therapy alone (circles); (b) human IVIg followed by the AAV gene therapy (squares); or (c) IVIg with IdeS followed by the AAV gene therapy. The level of AAV in the serum of the mice was measured by qPCR up to 7 days following administration of the AAV gene therapy and shown in Fig. 1.
**Fig 2****. qPCR detection of AAV in SCID Mice plasma up to 48 hours after AAV injection.** Shows results from the same experiment as Figure 1, but with serum AAV levels only shown up to 48 hours.
**Fig. 3****. qPCR detection of AAV in SCID Mice plasma at 1h, 4 hrs, 24 hrs and 48 hrs after AAV injection.** Shows results from the same experiment as Figures 1 and 2, but with serum AAV levels shown in a bar chart at the stated time-points.
**Fig. 4****. qPCR detection of AAV in SCID liver cells 14 days after AAV injection**. Shows results from the same experiment as Figures 1 to 3, but with AAV levels in liver cells being measured 14 days after administration of the AAV.
**Fig. 5****. qPCR detection of AAV in SCID heart cells 14 days after AAV injection**. Shows results from the same experiment as Figures 1 to 4, but with AAV levels in heart cells being measured 14 days after administration of the AAV.

### Brief Description of the Sequences

SEQ ID NO: 1 is the full sequence of IdeS including N terminal methionine and signal sequence. It is also available as NCBI Reference sequence no. WP_010922160.1
SEQ ID NO: 2 is the mature sequence of IdeS, lacking the N terminal methionine and signal sequence. It is also available as Genbank accession no. ADF13949.1
SEQ ID NO: 3 is the full sequence of IdeZ including N terminal methionine and signal sequence. It is also available as NCBI Reference sequence no. WP_014622780.1.
SEQ ID NO: 4 is the mature sequence of IdeZ, lacking the N terminal methionine and signal sequence.
SEQ ID NO: 5 is the sequence of a hybrid IdeS/Z. The N terminus is based on IdeZ lacking the N terminal methionine and signal sequence.
SEQ ID NOs: 6 to 25 are the sequences of exemplary proteases for use in the methods of the invention.
SEQ ID NO: 26 is the sequence of an IdeS polypeptide. Comprises the sequence of SEQ ID NO: 2 with an additional N terminal methionine and a histidine tag (internal reference pCART124).
SEQ ID NO: 27 is the sequence of an IdeZ polypeptide. Comprises the sequence of SEQ ID NO: 4 with an additional N terminal methionine and a histidine tag (internal reference pCART144).
SEQ ID NO: 28 is the sequence of an IdeS/Z polypeptide. Comprises the sequence of SEQ ID NO: 5 with an additional N terminal methionine and a histidine tag (internal reference pCART145).
SEQ ID NO: 29 is the contiguous sequence PLTPEQFRYNN, which corresponds to positions 63-73 of SEQ ID NO: 3.
SEQ ID NO: 30 is the contiguous sequence PPANFTQG, which corresponds to positions 58-65 of SEQ ID NO: 1.
SEQ ID NO: 31 is the contiguous sequence DDYQRNATEAYAKEVPHQIT, which corresponds to positions 35-54 of SEQ ID NO: 3.
SEQ ID NO: 32 is the contiguous sequence DSFSANQEIRYSEVTPYHVT, which corresponds to positions 30-49 of SEQ ID NO: 1.
SEQ ID NOs: 33 to 55 are nucleotide sequences encoding proteases set out above.
SEQ ID NOs: 56 to 69 are the sequences of exemplary proteases for use in the methods of the invention.
SEQ ID NO: 70 is the contiguous sequence NQTN, which corresponds to positions 336-339 of SEQ ID NO: 1.
SEQ ID NO: 71 is the contiguous sequence DSFSANQEIR YSEVTPYHVT, which corresponds to positions 30-49 of SEQ ID NO: 1.
SEQ ID NOs: 72 to 86 are nucleotide sequences encoding polypeptides disclosed herein.
SEQ ID NO: 87 is the sequence SFSANQEIRY SEVTPYHVT, which corresponds to positions 31-49 of SEQ ID NO: 1.
SEQ ID NO: 88 is the sequence DYQRNATEAY AKEVPHQIT, which corresponds to positions 36-54 of the IdeZ polypeptide NCBI Reference Sequence no WP_014622780.1.
SEQ ID NO: 89 is the sequence DDYQRNATEA YAKEVPHQIT, which may be present at the N terminus of a polypeptide of the invention.
SEQ ID NO: 90 is the mature sequence of EndoS (Endoglycosidase of S. pyogenes).
SEQ ID NO: 91 and SEQ ID NO: 92 are further exemplary proteases for use in the methods of the invention. SEQ ID NO: 92 is the same as SEQ ID NO: 91, except it lacks the first twenty residues at the N terminus of SEQ ID NO: 1 consisting of the contiguous sequence DDYQRNATEAY AKEVPHQIT.
SEQ ID NO: 93 is the amino acid sequence of the Spk1 protein.
SEQ ID NO: 94 is the amino acid sequence of the Spk2 protein.

### Detailed Description of the Invention

### General

It is to be understood that different applications of the disclosed products and methods may be tailored to the specific needs in the art. It is also to be understood that the terminology used herein is for the purpose of describing particular embodiments of the invention only, and is not intended to be limiting.

In addition as used in this specification and the appended claims, the singular forms "a", "an", and "the" include plural referents unless the content clearly dictates otherwise. Thus, for example, reference to "a polypeptide" includes "polypeptides", and the like.

A "polypeptide" is used herein in its broadest sense to refer to a compound of two or more subunit amino acids, amino acid analogs, or other peptidomimetics. The term "polypeptide" thus includes short peptide sequences and also longer polypeptides and proteins. As used herein, the term "amino acid" refers to either natural and/or unnatural or synthetic amino acids, including both D or L optical isomers, and amino acid analogs and peptidomimetics.

The terms "patient" and "subject" are used interchangeably and typically refer to a human. References to IgG typically refer to human IgG unless otherwise stated.

Where the term "comprising" is used herein, the invention also provides embodiments "consisting essentially of' and "consisting of' what is set out.

Amino acid identity as discussed above may be calculated using any suitable algorithm. For example the PILEUP and BLAST algorithms can be used to calculate identity or line up sequences (such as identifying equivalent or corresponding sequences (typically on their default settings), for example as described in Altschul S. F. (1993) J Mol Evol 36:290-300; Altschul, S, F et al (1990) J Mol Biol 215:403-10. Software for performing BLAST analyses is publicly available through the National Center for Biotechnology Information This algorithm involves first identifying high scoring sequence pairs (HSPs) by identifying short words of length W in the query sequence that either match or satisfy some positive-valued threshold score T when aligned with a word of the same length in a database sequence. T is referred to as the neighborhood word score threshold (Altschul *et al,* supra). These initial neighborhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program uses as defaults a word length (W) of 11, the BLOSUM62 scoring matrix (see Henikoff and Henikoff (1992) Proc. Natl. Acad. Sci. USA 89: 10915-10919) alignments (B) of 50, expectation (E) of 10, M=5, N=4, and a comparison of both strands.

The BLAST algorithm performs a statistical analysis of the similarity between two sequences; see e.g., Karlin and Altschul (1993) Proc. Natl. Acad. Sci. USA 90: 5873-5787. One measure of similarity provided by the BLAST algorithm is the smallest sum probability (P(N)), which provides an indication of the probability by which a match between two polynucleotide or amino acid sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001. Alternatively, the UWGCG Package provides the BESTFIT program which can be used to calculate identity (for example used on its default settings) (Devereux et al (1984) Nucleic Acids Research 12, 387-395).

All publications, patents and patent applications cited herein, whether supra or infra, are hereby incorporated by reference in their entirety.

### Enzyme treatment for enhancing gene therapy

The present invention provides a way to enhance the efficiency of gene therapy. For example, by using an enzyme able to degrade or digest and/or inhibit or reduce effector function of serum antibodies in the subject the half-life of a gene therapy may be extended. It may be that by using such an enzyme the dose of gene therapy agent administered is less than would have been usually administered in the absence of the enzyme. In some cases, it may be that the use of such an enzyme is able to enhance gene therapy for a subject even where the subject has only low or no such antibodies. It may be that the exposure of the subject to the gene therapy is increased and/or extended.

The present invention therefore provides a method of treating a subject in need of gene therapy for a disease comprising:
a. administering to said subject an amount of an enzyme effective to degrade or digest and/or inhibit or reduce effector function of serum antibodies in the subject; and
b. administering the gene therapy to said subject;

wherein the reaction products resulting from the activity of the enzyme prolong the serum half-life of the gene therapy in the subject and/or increase or prolong the exposure of the subject to the gene therapy;
wherein the gene therapy is administered at a dose which is lower than the minimally effective dose that would be administered to a subject in the absence of step a.; and
wherein said subject has no detectable antibodies that specifically bind to the gene therapy, or has either a low titre of neutralising antibodies that specifically bind to the gene therapy (NAb) or a low titre of total antibodies that specifically bind to the gene therapy (TAb), optionally wherein the presence/titre of antibodies in the subject is measured in a serum sample obtained from the subject prior to step a.

It may be that steps a. and b. are only carried out once each when the method is performed. It may be though that steps a. and b. are performed more than once, for example two or more times. In one case they are carried out three, four, five or more times. It may be that every time a subject requires gene therapy, the method outlined above is performed.

Steps a. and b. may be, for example, performed typically in the order set out, so step a. first and then step b. In one case step a. and step b. are performed from hours to one month apart. It may be that they are performed from hours to a fortnight apart. It may be that they are performed from one week to 4 hours apart. In one case, they are performed from 4 hour to 48 hours apart. In one preferred case, they are performed from 4 to 24 hours apart. In a further preferred case they are performed from 4 to 12 hours apart. It may be though that in some cases the two steps are performed at the same time. In one embodiment, the two are performed simultaneously, separately, or sequentially. Typically, the two are provided in separate compositions. In one embodiment though, the enzyme and gene therapy agent are provided in the same composition. In another embodiment, the enzyme may also be administered after the gene therapy as well, for instance up to any of the time periods mentioned above, but after administration of the gene therapy.

In one embodiment, after step a. measuring may be performed to determine when to perform step b), for example the level of antibody specific for the gene therapy may be measured. In one case, NAb may be measured. In another case, TAb may be measured. In one case both may be. In another case, such measurement may be performed prior to step a. to determine whether the subject is one that has no detectable antibodies that specifically bind to the gene therapy, or has either a low titre of NAb or a low titre of TAb, optionally wherein the presence/titre of antibodies in the subject is measured in a serum sample obtained from the subject prior to step a.

In some cases where a measurement is made it is made using a biological sample from the subject. A preferred biological sample is a blood product. In certain embodiments, a blood product comprises plasma or serum. A particularly preferred sample is serum.

The invention also provides a way to enhance the transduction efficiency of a gene therapy treatment.

The invention also provides a method for treating a subject not displaying a pre-existing ADA, or only having low ADA, specific for a gene therapy, the method comprising: a. administering to said subject an amount of an enzyme as described herein to the subject; and b. administering the gene therapy to said subject.

The present invention further provides a method for increasing the efficiency of a gene therapy, the method comprising: a. administering to said subject an amount of an enzyme as described herein to the subject; and b. administering the gene therapy to said subject, wherein at least part of the enhanced efficiency is separate to the impact of the treatment on any ADA present.

In one embodiment, the invention is applied to a subject whom previously would have only been given the gene therapy, but not the enzyme, because they did not display an ADA response.

The invention may be applied to any of the patient groups mentioned herein. It may be also employed as a way to enhance gene therapy for patients in general. For instance, an enzyme as discussed herein may be used to extend the clearance time of a gene therapy. An enzyme as discussed herein may be used to increase the exposure time of a patient to a gene therapy. It may be used to increase the overall exposure of a patient to a gene therapy, for instance in terms of the level of exposure and/or the duration of exposure. It may be used to increase the amount of gene therapy agent reaching a particular target organ. For instance, the invention may be used to increase the amount of a gene therapy agent reaching the liver. It may be used to increase the amount of a gene therapy agent reaching the heart. It may be used to extend the time the gene therapy agent is present in an organ such as those. The invention may be used to increase the time a gene therapy agent is present in the serum. It may be used to increase the level at which a gene therapy agent is present in the serum. In one embodiment, it may be the increased level of the gene therapy agent is seen at least at 48 hours after the administration of the agent, for instance at least at 24 hours after administration.

The present invention also provides a method of slowing the clearance of a gene therapy agent wherein the method provides
a. administering to said subject an amount of the enzyme effective to degrade or digest and/or inhibit or reduce effector function of serum antibodies in the subject; and
b. administering the gene therapy to said subject.

The gene therapy agent for instance may be any of those described herein. The enzyme may be, for instance, any of those described herein. In one embodiment, it may be that the rate of clearance is slower for at least the first 0 to 72 hours overall compared to if the enzyme had not been administered. In one embodiment, the rate of clearance may be slower for at least 0 to 48 hours after the gene therapy agent is administered. In one embodiment, the rate of clearance may be slower for at least 0 to 24 hours after the gene therapy agent is administered. In another, embodiment, it may be that the overall exposure of the subject to the gene therapy is increased for at least any of those time periods after the administration of the gene therapy agent, compared to if the enzyme was not also administered. In some embodiments, any of the timings of administration of the enzyme and/or gene therapy agent may be for any of the other embodiments described herein.

In another embodiment, the invention provides for the use of any of the antibody cleavage products of one of the enzymes set out herein to slow the rate of a gene therapy agent being cleared.

In an especially preferred embodiment, the rate of clearance or exposure is that in the serum of the subject.

### Enzyme

The enzyme employed is one typically able to degrade or digest and/or inhibit or reduce effector function of serum antibodies in the subject. The term "serum antibodies in the subject" may refer to any gamma immunoglobulin (IgG1, IgG2, IgG3 and IgG4) molecule which is present in human tissue or in circulation prior to a method of the invention being carried out.

The ability to degrade or digest and/or inhibit or reduce effector function of serum antibodies may mean a reduction in the Fc receptor interaction of IgG molecules. The term "Fc receptor" refers to Fc gamma immunoglobulin receptors, i.e. to FcyRs (Fc gamma receptors) which are present on cells. In humans, a Fcγ R refers to one, some, or all of the family of receptors comprising FcγRI (CD64), FcγRIIA (CD32A), FcγRIIB (CD32B), FcγRIIC (CD32C), FcγRIIIA (CD16a) and FcγRIIIB (CD16b). As used herein, the term Fcγ R includes naturally occurring polymorphisms of FcγRI (CD64), FcγRIIA (CD32A), FcγRIIB (CD32B), FcγRIIC (CD32C), FcγRIIIA (CD16a) and FcγRIIIB (CD16b).

The enzyme used in the method of the invention may be any enzyme which inactivates serum IgG, but is typically an IgG cysteine protease which cleaves IgG such that the antigen binding domains and Fc interacting domains are separated from each other. In such cases, Fc receptor interaction of serum IgG molecules is reduced because the quantity of intact IgG molecules in the serum is reduced. As another example, the enzyme may be an IgG endoglycosidase which cleaves a glycan structure on the Fc interacting domain of IgG, particularly the N-linked bi-antennary glycan at position Asn-297 (Kabat numbering). This glycan structure has a critical role in Fc receptor binding and complement activation. Thus, when it is wholly or partially removed by a protein, this will lead to reduced Fc receptor binding or complement activation by an otherwise intact IgG molecule, as well as also reduced recycling/half-life due to reduced binding to the FcRn. Enzymes suitable for use are discussed in more detail in subsequent sections below.

The enzyme is preferably administered by intravenous infusion. The enzyme though may be administered by any suitable route including, for example, intradermal, subcutaneous, percutaneous, intramuscular, intra-arterial, intraperitoneal, intraarticular, intraosseous or other appropriate administration routes. The amount of the enzyme that is administered may be between 0.01 mg/kg BW (BW - Body Weight) and 2 mg/kg BW, between 0.01 mg/kg BW and 1 mg/kg BW, between 0.01 mg/kg BW and 0.5 mg/kg BW, between 0.01 and 0.3 mg/kg BW, preferably between 0.1 and 0.3 mg/kg BW, most preferably between 0.2 and 0.3 mg/kg BW. In one case, the dose of enzyme is between 0.25 mg/kg BW and 0.5 mg/kg BW, preferably between 0.3 mg/kg BW and 0.5 mg/kg BW. A particularly preferred dose is approximately 0.25 mg/kg BW. The amount of enzyme administered will be typically enough to bring about the desired effect, for instance enough to extend the half-life of the gene therapy agent and/or to decrease the amount of the gene therapy agent needed to bring about a desired effect. The combination of the enzyme and the gene therapy may result in a synergistic effect. Hence, it may be that the amount of enzyme and the gene therapy is enough to bring about such a synergistic effect. The effect may be, for example, a decrease in the amount of the gene therapy agent needing to be administered. Alternatively, or additionally, it may be extending the half-life of the gene therapy agent. It may be that the half-life of the gene therapy agent is measured by qPCR. One preferred way to measure serum half-life is using qPCR to detect the gene therapy agent in the serum and/or plasma of the subject.

In a particularly preferred case, the dose of enzyme administered will be enough to degrade or digest and/or inhibit or reduce effector function of all or substantially all IgG molecules present in the serum of the subject, optionally wherein the enzyme is an IgG cysteine protease or an IgG endoglycosidase. By substantially all may be meant at least 90%. Preferably, substantially all means at least 95%. Even more preferably, substantially all means at least 99%.

The enzyme may be administered on multiple occasions to the same subject, provided that the quantity of anti-drug antibody (ADA) in the serum of the subject which is capable of binding to the enzyme does not exceed a threshold determined by the clinician. Preferably the enzyme will be administered prior to the gene therapy agent. For example, it may be that the enzyme is administered from 4 to 72 hours prior to the administration of the gene therapy agent. For example, it may be administered from 4 to 50 hours prior to administration of the gene therapy. Preferably, it may be that the enzyme is administered from 4 to 48 hours prior to administration of the gene therapy. More preferably, the enzyme may be administered from 4 to 6 hours prior to administration of the gene therapy agent. In one embodiment, the gene therapy is administered within 48 hours of the enzyme.

In some embodiments, the enzyme may be administered before and after the gene therapy. For example, it may be administered before, as explained above, but also within such times from administration of the gene therapy. Hence, for instance, it may be also administered within 48 hours of the gene therapy. It may be administered within 4 to 72 hours after the gene therapy. It may be administered from 4 to 6 hours after administration of the gene therapy.

In some embodiment, the enzyme and gene therapy may be administered in cycles. For instance, each cycle may comprise administration before the gene therapy with timing as set out above, as well as optionally after it. That may be repeated, for example, two three, four, five, or more times, with each repeat of administration of the enzyme and then the gene therapy considered a cycle.

In some embodiments, the invention may include, or the subject already have had performed, measurement of ADA. For example, it may be that the enzyme is given until the level of ADA is below a particular threshold.

The quantity of ADA in the serum of the subject which is capable of binding to the protease may be determined by any suitable method, such as an agent specific CAP FEIA (ImmunoCAP) test or a titer assay. If ADA in the subject exceed said threshold, the regimen may include administration of an alternative enzyme. In one embodiment, the assay employed may be a luciferase based assay. In another embodiment, the assay employed may be a bridging or a sandwich assay. In another embodiment, it may be a MSD assay.

Preferred enzymes include an IgG cysteine protease which is from a Streptococcus bacterium, such as *Streptococcus pyogenes, Streptococcus equi or Streptococcus zooepidemicus.* Particularly preferred enzymes include a IdeS, MAC2, SpeB, IdeZ or IgdE polypeptide.

Further preferred enzymes which may be employed include an IgG endoglycosidase. A preferred IgG endoglycosidase is one from a Streptococcus bacterium, such as Streptococcus pyogenes, Streptococcus equi or Streptococcus zooepidemicus, or from Corynebacterium pseudotuberculosis, Enterococcus faecalis, or Elizabethkingia meningoseptica. Particularly preferred enzymes are EndoS, CP40, EndoE, or EndoF2 polypeptide. An especially preferred enzyme is imlifidase. A further especially preferred enzyme is EndoS.

The enzyme may be any of the following:

### IgG cysteine proteases

The IgG cysteine protease for use with the invention is typically specific for IgG. In preferred embodiments, the protease for use in the methods of the invention is IdeS (Immunoglobulin G-degrading enzyme *of S. pyogenes*)*,* otherwise known as imlifidase. IdeS is an extracellular cysteine protease produced by the human pathogen *S. pyogenes.* IdeS was originally isolated from a group A *Streptococcus* strain of serotype M1, but the *ides* gene has now been identified in all tested group A *Streptococcus* strains. IdeS has an extraordinarily high degree of substrate specificity, with its only identified substrate being IgG. IdeS catalyses a single proteolytic cleavage in the lower hinge region of the heavy chains of all subclasses of human IgG. IdeS also catalyses an equivalent cleavage of the heavy chains of some subclasses of IgG in various animals. IdeS efficiently cleaves IgG to Fc and F(ab')₂ fragments via a two-stage mechanism. In the first stage, one (first) heavy chain of IgG is cleaved to generate a single cleaved IgG (scIgG) molecule with a non-covalently bound Fc/2 molecule. The scIgG molecule is effectively an intermediate product which retains the remaining (second) heavy chain of the original IgG molecule. In the second stage of the mechanism this second heavy chain is cleaved by IdeS to release a F(ab')₂ fragment and a homodimeric Fc fragment. These are the products generally observed under physiological conditions. Under reducing conditions the F(ab')₂ fragment may dissociate to two Fab' fragments and the homodimeric Fc may dissociate into its component monomers. IdeS has been shown to be particularly effective at cleaving IgG in humans. The entire plasma IgG-pool is cleaved within minutes of dosing with IdeS, and IgG levels in blood remain low for more than a week until newly synthesized IgG appeared in plasma. This demonstrates that the entire extracellular IgG pool and not only the plasma pool (i.e. serum IgG molecules) is cleaved by IdeS (Winstedt et al; PloS One 2015; 10(7): e0132011).

SEQ ID NO: 1 is the full sequence of IdeS including the N terminal methionine and signal sequence. It is also available as NCBI Reference sequence no. WP_010922160.1. SEQ ID NO: 2 is the mature sequence of IdeS, lacking the N terminal methionine and signal sequence. It is also available as Genbank accession no. ADF13949.1.

In alternative embodiments, the protease for use in the methods of the invention is IdeZ, which is a IgG cysteine protease produced by *Streptococcus equi ssp. Zooepidemicus,* a bacterium predominantly found in horses. SEQ ID NO: 3 is the full sequence of IdeZ including N terminal methionine and signal sequence. It is also available as NCBI Reference sequence no. WP_014622780.1. SEQ ID NO: 4 is the mature sequence of IdeZ, lacking the N terminal methionine and signal sequence.

In alternative embodiments, the protease for use in the methods of the invention is a hybrid IdeS/Z, such as that of SEQ ID NO: 5. The N terminus is based on IdeZ lacking the N terminal methionine and signal sequence.

In preferred embodiments, the protease for use in the invention may comprise or consist of SEQ ID NO: 2, 4 or 5. Proteases for use in the invention may comprise an additional methionine (M) residue at the N terminus and/or a tag at the C terminus to assist with expression in and isolation from standard bacterial expression systems. Suitable tags include a histidine tag which may be joined directly to the C terminus of a polypeptide or joined indirectly by any suitable linker sequence, such as 3, 4 or 5 glycine residues. The histidine tag typically consists of six histidine residues, although it can be longer than this, typically up to 7, 8, 9, 10 or 20 amino acids or shorter, for example 5, 4, 3, 2 or 1 amino acids.

In further preferred embodiments, the protease for use in the invention may comprise, consist essentially, or consist of the sequence of any one of SEQ ID NOs: 6 to 25. These sequences represent IdeS and IdeZ polypeptides with increased protease activity and/or reduced immunogenicity. Each of SEQ ID NOs: 6 to 25 may optionally include an additional methionine at the N terminus and/or a histidine tag at the C terminus. The histidine tag preferably consists of six histidine residues. The histidine tag is preferably linked to the C terminus by a linker of 3x glycine or 5x glycine residues.

In further preferred embodiments, the protease for use in the invention may comprise, consist essentially, or consist of the sequence of any one of SEQ ID NOs: 56 to 69. These sequences represent IdeS polypeptides with increased protease activity and/or reduced immunogenicity. Each of SEQ ID NOs: 56 to 69 may optionally include an additional methionine at the N terminus and/or a histidine tag at the C terminus. The histidine tag preferably consists of six histidine residues. The histidine tag is preferably linked to the C terminus by a linker of 3x glycine or 5x glycine residues.

In further preferred embodiments, the protease for use in the invention may comprise, consist essentially, or consist of the sequence of any one of SEQ ID NOs: 6 to 25, optionally with up to 3 (such as 1, 2 or 3) amino acid substitutions. Each of SEQ ID NOs: 6 to 25 and variants thereof may optionally include an additional methionine at the N terminus and/or a histidine tag at the C terminus.

In further preferred embodiments, the protease for use in the invention may comprise, consist essentially, or consist of the sequence of any one of SEQ ID NOs: 56 to 69, optionally with up to 3 (such as 1, 2 or 3) amino acid substitutions. Each of SEQ ID NOs: 56 to 69 and variants thereof may optionally include an additional methionine at the N terminus and/or a histidine tag at the C terminus.

The polypeptide of the invention is typically at least 100, 150, 200, 250, 260, 270, 280, 290, 300 or 310 amino acids in length. The polypeptide of the invention is typically no larger than 400, 350, 340, 330, 320 or 315 amino acids in length. It will be appreciated that any of the above listed lower limits may be combined with any of the above listed upper limits to provide a range for the length the polypeptide of the invention. For example, the polypeptide may be 100 to 400 amino acids in length, or 250 to 350 amino acids in length. The polypeptide is preferably 290 to 320 amino acids in length, most preferably 300 to 315 amino acids in length.

The primary structure (amino acid sequence) of a protease of the invention is based on the primary structure of IdeS, IdeZ or IdeS/Z, specifically the amino acid sequence of SEQ ID NO: 2, 4 or 5, respectively. The sequence of a protease of the invention may comprise a variant of the amino acid sequence of SEQ ID NO: 2, 4 or 5, which is at least 80% identical to the amino acid sequence of SEQ ID NO: 2, 4 or 5. The variant sequence may be at least 80%, at least, 85%, preferably at least 90%, at least 95%, at least 98% or at least 99% identical to the sequence of SEQ ID NO: 2, 4 or 5. The variant may be identical to the sequence of SEQ ID NO: 2, 4 or 5 apart from the inclusion of one or more of the specific modifications identified in WO2016/128558 or WO2016/128559. Identity relative to the sequence of SEQ ID NO: 2, 4 or 5 can be measured over a region of at least 50, at least 100, at least 200, at least 300 or more contiguous amino acids of the sequence shown in SEQ ID NO: 2, 4 or 5, or more preferably over the full length of SEQ ID NO: 4 or 5.

The protease for use in the invention may be an IdeS, IdeZ or IdeS/Z polypeptide that comprises a variant of the amino acid sequence of SEQ ID NO:, 2, 4 or 5 in which modifications, such as amino acid additions, deletions or substitutions are made relative to the sequence of SEQ ID NO: 2, 4 or 5. Such modifications are preferably conservative amino acid substitutions. Conservative substitutions replace amino acids with other amino acids of similar chemical structure, similar chemical properties or similar side-chain volume. The amino acids introduced may have similar polarity, hydrophilicity, hydrophobicity, basicity, acidity, neutrality or charge to the amino acids they replace. Alternatively, the conservative substitution may introduce another amino acid that is aromatic or aliphatic in the place of a pre-existing aromatic or aliphatic amino acid. Conservative amino acid changes are well-known in the art.

IgG cysteine protease activity may be assessed by any suitable method, for example by incubating a polypeptide with a sample containing IgG and determining the presence of IgG cleavage products. Suitable methods are described in the WO2016/128559. Suitable assays include an ELISA-based assay, such as that which is described in WO2016/128559. In such an assay, the wells of an assay plate will be typically coated with an antibody target, such as bovine serum albumin (BSA). Samples of the polypeptide to be tested are then added to the wells, followed by samples of target-specific antibody that is antibody specific for BSA in this example. The polypeptide and antibody are allowed to interact under conditions suitable for IgG cysteine protease activity. After a suitable interval, the assay plate will be washed and a detector antibody which specifically binds to the target-specific antibody will be added under conditions suitable for binding to the target-specific antibody. The detector antibody will bind to any intact target-specific antibody that has bound to the target in each well. After washing, the amount of detector antibody present in a well will be proportional to the amount of target-specific antibody bound to that well. The detector antibody may be conjugated directly or indirectly to a label or another reporter system (such as an enzyme), such that the amount of detector antibody remaining in each well can be determined. The higher the potency of the tested polypeptide that was in a well, the less intact target-specific antibody will remain and thus there will be less detector antibody. Typically, at least one well on a given assay plate will include IdeS instead of a polypeptide to be tested, so that the potency of the tested polypeptides may be directly compared to the potency of IdeS. IdeZ and IdeS/Z may also be included for comparison.

Other assays may determine the potency of a tested polypeptide by directly visualizing and/or quantifying the fragments of IgG which result from cleavage of IgG by a tested polypeptide. An assay of this type is also described in WO2016/128559. Such an assay will typically incubate a sample of IgG with a test polypeptide (or with one or more of IdeS, IdeZ and IdeS/Z as a control) at differing concentrations in a titration series. The products which result from incubation at each concentration are then separated using gel electrophoresis, for example by SDS-PAGE. Whole IgG and the fragments which result from cleavage of IgG can then be identified by size and quantified by the intensity of staining with a suitable dye. The greater the quantity of cleavage fragments, the greater the potency of a tested polypeptide at a given concentration. A polypeptide of the invention will typically produce detectable quantities of cleavage fragments at a lower concentration (a lower point in the titration series) than IdeZ and/or IdeS. This type of assay may also enable the identification of test polypeptides that are more effective at cleaving the first or the second heavy chain of an IgG molecule, as the quantities of the different fragments resulting from each cleavage event may also be determined. A polypeptide of the invention may be more effective at cleaving the first chain of an IgG molecule than the second, particularly when the IgG is an IgG2 isotype. A polypeptide of the invention may be more effective at cleaving IgG1 than IgG2.

In one particularly preferred case the IgG cysteine protease is a polypeptide comprising or consisting of a sequence that is at least 80% identical to SEQ ID NO: 2, 4, 5 such as at least 85%, 90%, 95%, 99% or 100% identical. In a further particularly preferred case the IgG cysteine protease comprises or consists of the sequence of any one of SEQ ID NOs: 6 to 25 and 55 to 69, 91 or 92. Optionally the sequence includes an additional methionine at the N terminus and/or a histidine tag at the C terminus.

### IgG endoglycosidases

The enzyme may have IgG endoglycosidase activity, preferably cleaving the glycan moiety at Asn-297 (Kabat numbering) in the Fc region of IgG. An example of such a protein is EndoS (Endoglycosidase of *S. pyogenes*)*.* EndoS hydrolyzes the β-1,4-di-*N-*acetylchitobiose core of the asparagine-linked glycan of normally-glycosylated IgG. The mature sequence of EndoS is provided as SEQ ID NO: 90. The agent may be a protein comprising or consisting of the amino acid sequence of SEQ ID NO: 90, or may be a homologue thereof from an alternative bacterium, such as Streptococcus equi or Streptococcus zooepidemicus, or Corynebacterium pseudotuberculosis, Enterococcus faecalis, or Elizabethkingia meningoseptica. The agent may be CP40, EndoE, or EndoF₂.

Alternatively the agent may be a variant of the EndoS protein which comprises or consists of any amino acid sequence which has at least 80%, 85%, 90% or 95% identity with SEQ ID NO: 90 and has IgG endoglycosidase activity. A variant of the EndoS protein may comprise or consist of an amino acid sequence in which up to 1, 2, 3, 4, 5, 10, 20, 30, 40, 50, 60, 70, 80, 90, or more, amino acid substitutions, insertions or deletions have been made relative to the amino acid sequence of SEQ ID NO: 90, provided the variant has IgG endoglycosidase activity. Said amino acid substitutions are preferably conservative. Conservative substitutions are as defined in the preceding section.

Alternatively the agent may be a protein which comprises or consists of a fragment of SEQ ID NO: 90 and has IgG endoglycosidase activity, preferably wherein said fragment is 400 to 950, 500 to 950, 600 to 950, 700 to 950 or 800 to 950 amino acids in length. A preferred fragment consists of amino acids 1 to 409 of SEQ ID NO: 90, which corresponds to the enzymatically active α-domain of EndoS generated by cleavage by the streptococcal cysteine proteinase SpeB. The fragment may be created by the deletion of one or more amino acid residues of the amino acid sequence of SEQ ID NO: 90. Up to 1, 2, 3, 4, 5, 10, 20, 30, 40, 50, 100, 200, 300, 400, 500 or 550 residues may be deleted, or more. The deleted residues may be contiguous with other.

Any fragment or variant of SEQ ID NO: 90 preferably includes residues 191 to 199 of SEQ ID NO: 90, i.e. Leu-191, Asp-192, Gly-193, Leu-194, Asp-195, Val-196, Asp-197, Val-198 and Glu-199 of SEQ ID NO: 90. These amino acids constitute a perfect chitinase family 18 active site, ending with glutamic acid. The glutamic acid in the active site of chitinases is essential for enzymatic activity. Most preferably, therefore, a variant of SEQ ID NO: 90 contains Glu-199 of SEQ ID NO: 90. The variant of SEQ ID NO: 90 may contain residues 191 to 199 of SEQ ID NO: 90 having one or more conservative substitutions, provided that the variant contains Glu-199 of SEQ ID NO: 90.

### Gene therapy agents

A gene therapy for use in the present invention may be any suitable gene therapy. It may be a gene therapy agent to treat any of the conditions mentioned herein. For example, a gene therapy agent may be one comprise a nucleic acid encoding a therapeutic gene of interest and means for its expression. It may be that the therapeutic gene is able to compensate for a non-functional or defective gene, for example such a gene in any of the conditions mentioned herein. The gene therapy may be, for example, a gene augmentation, gene inhibition or suicide gene therapy. Preferably it is a gene augmentation therapy.

Preferably, the gene therapy is a viral gene therapy. An example of an especially preferred viral gene therapy is an AAV (Adeno-associated virus) gene therapy. Further examples of possible gene therapy vectors include adenoviral vectors. Other examples of gene therapy vectors include lentiviral and retroviral gene therapy vectors. Further examples of gene therapy vectors include CRISPR based gene therapy vectors.

In one case the gene therapy agent comprises a lentiviral vector which comprises envelope proteins to which antibodies of the subject specifically bind. In a preferred case, the gene therapy comprises a AAV vector which comprises capsid proteins to which antibodies of the subject specifically bind. Optionally, antibodies from the subject specifically bind VP1, VP2 and/or VP3 capsid proteins.

Examples of AAV vectors that may be employed include, for example, AAV type 1, AAV type 2, AAV type 3 (e.g. types 3 A and 3B), AAV type 4, AAV type 5, AAV type 6, AAV type 7, AAV type 8, AAV type 9, AAV type 10, AAV type 11, AAV type 12, AAV type 13, AAV type rh32.33, AAV type rh8, AAV type rhlO, AAV type rh74, AAV type hu.68, avian AAV, bovine AAV, canine AAV, equine AAV, ovine AAV, snake AAV, bearded dragon AAV, AAV2i8, AAV2g9, AAV-LK03, AAV7m8, AAV Anc80, AAV PHP.B, and any other AAV now known or later discovered. See, e.g., BERNARD N. FIELDS et al, VIROLOGY, volume 2, chapter 69 (4th ed., Lippincott-Raven Publishers). A number of AAV serotypes and clades have been identified (see, e.g., Gao et al, (2004) J. Virology 78:6381-6388; Moris et al, (2004) Virology 33-:375-383). The AAV employed may be a chimeric AAV, that is an AAV comprising a capsid protein with regions, domains, individual amino acids that are derived from two or more different serotypes of AAV.

In one embodiment, the said AAV vector comprises VP1, VP2 and/or VP3 capsid protein having at least 60%, 70%, 80%, 90%, 95% or 100% sequence identity to VP1, VP2 and/or VP3 capsid protein selected from the group consisting of AAV1 , AAV2, AAV3, AAV4, AAV5, AAV6, AAV7, AAV8, AAV9, AAV10, AAV3B, AAV-2i8, RhlO, Rh74, SEQ ID NO: 93 and SEQ ID NO: 94, VP1 , VP2 and/or VP3 capsid proteins.

The gene therapy may be administered at a suitable dose. One advantage of the present invention is that the use of the enzyme will typically result in a reduction in the amount of the gene therapy agent needed to be given compared to that usually given in the absence of the enzyme. The enzyme and gene therapy therefore represent a synergistic combination. Examples of the reduction seen include a reduction of at least 5%. In one case the reduction is at least 10%. In another the reduction is at least 20%. For example, in some cases the reduction is at least 25%, 30%, 40%, at least 50%, or greater. In other examples the reduction seen is in a range formed by any two of those values. In an alternative embodiment, rather than a reduced dose being given the same dose is given but with a bigger effect seen for the gene therapy, for example the level of expression may be increased by at least any of the percentage values mentioned in this paragraph.

In one preferred embodiment, the gene therapy agent is a viral vector which is administered at a dose which is lower than 1 × 10¹⁵ vector genomes (vg)/kg body weight (vg/kg). In some cases, it may be that dose is lower than 10¹⁴ vg/kg body weight. In some cases, it may be that dose is lower than 10¹³ vg/kg body weight. In some preferred cases it may be that dose is lower than 10¹² vg/kg body weight. In some more preferred cases it may be that dose is lower than 10¹¹ vg/kg body weight. In some even more preferred cases it may be that the dose is lower than 1 × 10¹⁰ vg/kg, lower than 1 × 10⁹ vg/kg, lower than 1 × 10⁸ vg/kg, or lower than 0.99 × 10⁷ vg/kg. It may be that the invention leads to a reduction in the dosage to be administered to one of those levels. It may be that the dosage to be given, but still be effective, can be reduced to a level that a subject previously precluded from gene therapy is eligible.

In another embodiment, the gene therapy agent may be a non-viral vector. For example, it may be that the gene therapy agent is selected from the group consisting of liposomes, nanoparticles, lipid nanoparticles, polymers, microparticles, microcapsules, micelles, or extracellular vesicles.

It may be that the invention leads to increased exposure of a subject to the gene therapy agent. It may be that the length of exposure is increased. It may be that the exposure at a given time point is greater when the invention is employed. It may be that the overall exposure in terms of the level and length of exposure is increased. It may be that the invention leads to the level of the gene therapy reaching a given target organ is increased. It may be that the overall length of exposure to the gene therapy agent is increased. The level of any such increases may be, for instance, at least 5%. It may be at least 10%. It may be at least 25%. It may be at least 50%. It may be at least 75%. It may be at least 100%. It may be the increase seen is a range with two of those values as endpoints.

The present invention provides for the use of an enzyme as described herein to achieve any of the benefits specified herein, wherein the subject is also administered the gene therapy.

### Diseases and conditions to be treated

Gene therapy may be used to treat potentially almost any disease or disorder.

In one preferred case the disease to be treated is one caused by a loss of function or activity of a protein, and the gene therapy comprises a heterologous polynucleotide that encodes a protein or peptide that when expressed in the subject provides or supplements a function or activity of the protein. In a further preferred case the disease to be treated is one caused by a gain of function activity or expression of a protein, and the gene therapy comprises a heterologous polynucleotide that is transcribed into a nucleic acid that inhibits, decreases or reduces expression of the gain of function, activity or expression of said protein.

Preferred diseases and disorders which may be treated using the invention include proliferative diseases (cancers, tumors, dysplasias, etc.), Crigler-Najjar and metabolic diseases like metabolic diseases of the liver, Friedreich ataxia, infectious diseases, addiction (e.g ., to tobacco, alcohol, or drugs), epilepsy, Canavan's disease, adrenoleukodystrophy, viral diseases (induced, e.g., by hepatitis B or C viruses, HIV, herpes, retroviruses, etc.), genetic diseases (cystic fibrosis, dystroglycanopathies, myopathies such as Duchenne muscular myopathy or dystrophy, myotubular myopathy, hemophilia A, hemophilia B, sickle-cell anemia, sickle cell disease, Fanconi's anemia, diabetes, amyotrophic lateral sclerosis (AFS), myotubularin myopathy, motor neuron diseases such as spinal muscular atrophy (SMA), spinobulbar muscular atrophy, or Charcot-Marie-Tooth disease, arthritis, severe combined immunodeficiencies (such as RS- SCID, ADA-SCID or X-SCID), Wiskott-Aldrich syndrome, X-linked thrombocytopenia, X-linked congenital neutropenia, chronic granulomatous disease, etc.), clotting factor deficiencies, cardiovascular disease (restenosis, ischemia, dyslipidemia, homozygous familial hypercholesterolemia, etc.), eye diseases such as retinitis pigmentosa, Feber congenital amaurosis, Feber hereditary optic neuropathy, and Stargardt disease; lysosomal storage diseases such as San Filippo syndrome; hyperbilirubinemia such as CN type I or II or Gilbert's syndrome; Fabry disease, glycogen storage disease such as GSDI, GSDII (Pompe disease), GSDIII, GSDIV, GSDV, GSDVI, GSDVII, GSDVIII and lethal congenital glycogen storage disease of the heart.

In one embodiment, the subject to be treated has:
- a lung disease (e.g ., cystic fibrosis); a bleeding disorder (e.g., hemophilia A or hemophilia B with or without inhibitors), thalassemia, a blood disorder (e.g., anemia), Alzheimer's disease, Parkinson's disease, Huntington's disease, amyotrophic lateral sclerosis (ALS), epilepsy, a lysosomal storage disease (e.g., aspartylglucosaminuria), Batten disease, late infantile neuronal ceroid lipofuscinosis type 2 (CLN2), cystinosis, Fabry disease, Gaucher disease types I, II, and III, glycogen storage disease II (Pompe disease), GM2-gangliosidosis type I (Tay Sachs disease), GM2-gangliosidosis type II (Sandhoff disease), mucolipidosis types I (sialidosis type I and II), II (I-cell disease), III (pseudo-Hurler disease) and IV, mucopolysaccharide storage diseases (Hurler disease and variants, Hunter, Sanfilippo Types A,B,C,D, Morquio Types A and B, Maroteaux-Lamy and Sly diseases), Niemann-Pick disease types A/B, C1 and C2, and Schindler disease types I and II), hereditary angioedema (HAE), a copper or iron accumulation disorder (e.g., Wilson's or Menkes disease), lysosomal acid lipase deficiency, a neurological or neurodegenerative disorder, cancer, type 1 or type 2 diabetes, adenosine deaminase deficiency, a metabolic defect (e.g., glycogen storage diseases), a disease of solid organs (e.g., brain, liver, kidney, heart), or an infectious viral (e.g., hepatitis B and C, HIV, etc.), bacterial or fungal disease;
- a blood clotting disorder, optionally hemophilia A, hemophilia A with inhibitory antibodies, hemophilia B, hemophilia B with inhibitory antibodies, a deficiency in any coagulation Factor: VII, VIII, IX, X, XI, V, XII, II, von Willebrand factor, or a combined FV/FVIII deficiency, thalassemia, vitamin K epoxide reductase Cl deficiency or gamma-carboxylase deficiency;
- anemia, bleeding associated with trauma, injury, thrombosis, thrombocytopenia, stroke, coagulopathy, disseminated intravascular coagulation (DIC); over-anticoagulation associated with heparin, low molecular weight heparin, pentasaccharide, warfarin, small molecule antithrombotics (i.e., FXa inhibitors), or a platelet disorder such as, Bernard Soulier syndrome, Glanzmann thrombasthenia, or storage pool deficiency.

In another preferred embodiment, the gene therapy is one that comprises inhibitory nucleic acid binds to a gene, a transcript of a gene, or a transcript of a gene associated with a polynucleotide repeat disease selected from the group consisting of a huntingtin (HTT) gene, a gene associated with dentatorubropallidoluysian atrophy (atrophin 1, ATN1), androgen receptor on the X chromosome in spinobulbar muscular atrophy, human Ataxin-1, -2, -3, and -7, Ca v 2.1 P/Q voltage-dependent calcium channel (CACNA1A), TATA-binding protein, Ataxin 8 opposite strand (ATXN80S), Serine/threonine-protein phosphatase 2A 55 kDa regulatory subunit B beta isoform in spinocerebellar ataxia (type 1, 2, 3, 6, 7, 8, 12 17), FMR1 (fragile X mental retardation 1) in fragile X syndrome, FMR1 (fragile X mental retardation 1) in fragile X-associated tremor/ataxia syndrome, FMR1 (fragile X mental retardation 2) or AF4/FMR2 family member 2 in fragile XE mental retardation; Myotonin-protein kinase (MT-PK) in myotonic dystrophy; Frataxin in Friedreich's ataxia; a mutant of superoxide dismutase 1 (SOD1) gene in amyotrophic lateral sclerosis; a gene involved in pathogenesis of Parkinson's disease and/or Alzheimer's disease; apolipoprotein B (APOB) and proprotein convertase subtilisin/kexin type 9 (PCSK9), hypercholesterolemia; HIV Tat, human immunodeficiency virus transactivator of transcription gene, in HIV infection; HIV TAR, HIV TAR, human immunodeficiency virus transactivator response element gene, in HIV infection; C-C chemokine receptor (CCR5) in HIV infection; Rous sarcoma virus (RSV) nucleocapsid protein in RSV infection, liver-specific microRNA (miR-122) in hepatitis C virus infection; p53, acute kidney injury or delayed graft function kidney transplant or kidney injury acute renal failure; protein kinase N3 (PKN3) in advance recurrent or metastatic solid malignancies; LMP2, LMP2 also known as proteasome subunit beta-type 9 (PSMB 9), metastatic melanoma; LMP7,also known as proteasome subunit beta-type 8 (PSMB 8), metastatic melanoma; MECL1 also known as proteasome subunit beta-type 10 (PSMB 10), metastatic melanoma; vascular endothelial growth factor (VEGF) in solid tumors; kinesin spindle protein in solid tumors, apoptosis suppressor B- cell CLL/lymphoma (BCL-2) in chronic myeloid leukemia; ribonucleotide reductase M2 (RRM2) in solid tumors; Furin in solid tumors; polo-like kinase 1 (PLK1) in liver tumors, diacylglycerol acyltransferase 1 (DGAT1) in hepatitis C infection, beta-catenin in familial adenomatous polyposis; beta2 adrenergic receptor, glaucoma; RTP801/Reddl also known as DNA damage-inducible transcript 4 protein, in diabetic macular edema (DME) or age- related macular degeneration; vascular endothelial growth factor receptor I (VEGFR1) in age-related macular degeneration or choroidal neovascularization, caspase 2 in non- arteritic ischaemic optic neuropathy; Keratin 6 A N 17K mutant protein in pachyonychia congenital; influenza A virus genome/gene sequences in influenza infection; severe acute respiratory syndrome (SARS) coronavirus genome/gene sequences in SARS infection; respiratory syncytial virus genome/gene sequences in respiratory syncytial virus infection; Ebola filovirus genome/gene sequence in Ebola infection; hepatitis B and C virus genome/gene sequences in hepatitis B and C infection; herpes simplex virus (HSV) genome/gene sequences in HSV infection, coxsackievirus B3 genome/gene sequences in coxsackievirus B3 infection; silencing of a pathogenic allele of a gene (allele-specific silencing) like torsin A (TORI A) in primary dystonia, pan-class I and HLA-allele specific in transplant; and mutant rhodopsin gene (RHO) in autosomal dominantly inherited retinitis pigmentosa (adRP); and wherein said inhibitory nucleic acid is optionally a siRNA, an antisense molecule, miRNA, RNAi, a ribozyme or a shRNA.

The gene therapy agent may be one that leads to expression of a missing or deficient protein in a subject. For example, the invention may be used in treating a growth hormone deficiency. It may be used to treat an immunodeficiency. In another case, the gene therapy agent may be one that leads to killing of cells involved in the underlying pathology of the disease state. Further examples of diseases which may be treated include sickle cell anemia, SCID, CF, haemophilia, DMD, Huntington's, Parkinson's, hypercholesterolemia, alpha-trypsin deficiency, CGD, Faconi anemia and Gaucher's disease.

The gene therapy will be typically a treatment where the gene therapy agent is administered directly to the subject. Treating a disorder or condition as specified herein includes both preventing and treating the disorder. It may include ameliorating or improving one or more symptoms of the disorder to the condition as a whole.

The invention also provides an enzyme which inactivates serum antibodies for use in a method for the prevention or treatment of a disease or condition, wherein the method is as described above. The invention also provides the use of an enzyme which inactivates serum antibodies in a subject in the manufacture of a medicament, wherein the medicament is for the prevention or treatment of a disease or condition in a method as described above.

The invention also provides a gene therapy agent for use in a method for the prevention or treatment of a disease or condition, wherein the method is as described above. The invention also provides a gene therapy agent in the manufacture of a medicament, wherein the medicament is for the prevention or treatment of a disease or condition in a method as described above.

### Subjects to be treated

In terms of the subject to be treated, they will typically have one of the conditions mentioned herein. One advantage of the present invention is also that it is effective even where the subject does not have antibodies against the gene therapy agent or only has lower levels of such antibodies. Hence, in one case it may be that the subject has no detectable antibodies that specifically bind to the gene therapy. In another, it may be that the subject has a low titre of neutralising antibodies that specifically bind to the gene therapy (NAb). A neutralizing antibody may be one, for example, that prevents the gene therapy agent binding its target or target cell. In one embodiment, the subject may have a titre of NAb of <1:10 or a TAb titre of <1:100. It may be that the titre of NAb is <1:50, <1:100, <1:500 or <1:1000. It may be that the TAb titre is <1:500, <1:1000, <1:10,000 or less.

Owing to the invention enhancing the effect of the gene therapy, it may be that the subject is one who could not previously have been treated by the gene therapy because the effect was not big enough.

In other embodiments, the invention may be applied to patients in general, not just specific patient groups. The invention helps improve gene therapy and so may be applied to any patient which would benefit from the gene therapy. For instance, the ability of the invention to prolong exposure of a subject to a gene therapy agent may be used for gene therapy patients in general, not just those with low, or no, serum antibodies.

The subject to be treated is typically a mammal. In an especially preferred case, the subject to be treated is human.

In one case, the subject may be a subject that has not previously received gene therapy. It may be that the subject is one that who has not previously received the gene therapy that is to be given as part of the method.

### Compositions and formulations comprising polypeptides

The present invention also provides compositions comprising an enzyme for use in the methods of the invention. For example, the invention provides a composition comprising one or more polypeptides, and at least one pharmaceutically acceptable carrier or diluent. The present invention also provides compositions comprising a gene therapy agent for use in the methods of the invention. For example, the invention provides a composition comprising a gene therapy agent, and at least one pharmaceutically acceptable carrier or diluent.

The carrier(s) must be 'acceptable' in the sense of being compatible with the other ingredients of the composition and not deleterious to a subject to which the composition is administered. Typically, carriers and the final composition are sterile and pyrogen free.

Formulation of a suitable composition can be carried out using standard pharmaceutical formulation chemistries and methodologies all of which are readily available to the reasonably skilled artisan. For example, the enzyme can be combined with one or more pharmaceutically acceptable excipients or vehicles. Auxiliary substances, such as wetting or emulsifying agents, pH buffering substances, reducing agents and the like, may be present in the excipient or vehicle. Suitable reducing agents include cysteine, thioglycerol, thioredoxin, glutathione and the like. Excipients, vehicles and auxiliary substances are generally pharmaceutical agents that do not induce an immune response in the individual receiving the composition, and which may be administered without undue toxicity. Pharmaceutically acceptable excipients include, but are not limited to, liquids such as water, saline, polyethylene glycol, hyaluronic acid, glycerol, thioglycerol and ethanol. Pharmaceutically acceptable salts can also be included therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients, vehicles and auxiliary substances is available in Remington's Pharmaceutical Sciences (Mack Pub. Co., N.J. 1991).

Such compositions may be prepared, packaged, or sold in a form suitable for bolus administration or for continuous administration. Injectable compositions may be prepared, packaged, or sold in unit dosage form, such as in ampoules or in multi-dose containers containing a preservative. Compositions include, but are not limited to, suspensions, solutions, emulsions in oily or aqueous vehicles, pastes, and implantable sustained-release or biodegradable formulations. Such compositions may further comprise one or more additional ingredients including, but not limited to, suspending, stabilizing, or dispersing agents. In one embodiment of a composition for parenteral administration, the active ingredient is provided in dry (for e.g., a powder or granules) form for reconstitution with a suitable vehicle (e. g., sterile pyrogen-free water) prior to parenteral administration of the reconstituted composition. The compositions may be prepared, packaged, or sold in the form of a sterile injectable aqueous or oily suspension or solution. This suspension or solution may be formulated according to the known art, and may comprise, in addition to the active ingredient, additional ingredients such as the dispersing agents, wetting agents, or suspending agents described herein. Such sterile injectable formulations may be prepared using a non-toxic parenterally-acceptable diluent or solvent, such as water or 1,3-butane diol, for example. Other acceptable diluents and solvents include, but are not limited to, Ringer's solution, isotonic sodium chloride solution, and fixed oils such as synthetic mono-or di-glycerides.

Other parenterally-administrable compositions which are useful include those which comprise the active ingredient in microcrystalline form, in a liposomal preparation, or as a component of a biodegradable polymer systems. Compositions for sustained release or implantation may comprise pharmaceutically acceptable polymeric or hydrophobic materials such as an emulsion, an ion exchange resin, a sparingly soluble polymer, or a sparingly soluble salt. The compositions may be suitable for administration by any suitable route including, for example, intradermal, subcutaneous, percutaneous, intramuscular, intra-arterial, intraperitoneal, intraarticular, intraosseous or other appropriate administration routes. Preferred compositions are suitable for administration by intravenous infusion.

A composition of the invention will typically provide an "effective amount" or "sufficient amount" refers to an amount that provides, in single or multiple doses, alone or in combination, with one or more other compositions, treatments, protocols, or therapeutic regimens agents, a detectable response of any duration of time (long or short term), an expected or desired outcome in or a benefit to a subject of any measurable or detectable degree or for any duration of time (e.g., for minutes, hours, days, months, years, or cured). The doses of an "effective amount" or "sufficient amount" for treatment (e.g., to ameliorate or to provide a therapeutic benefit or improvement) typically are effective to provide a response to one, multiple or all adverse symptoms, consequences or complications of the disease, one or more adverse symptoms, disorders, illnesses, pathologies, or complications, for example, caused by or associated with the disease, to a measurable extent, although decreasing, reducing, inhibiting, suppressing, limiting or controlling progression or worsening of the disease is a satisfactory outcome.

### Kits

The invention also provides a kit for carrying out the methods described herein. The kit of the invention may include an enzyme or a composition comprising an enzyme, as described above. The kit may include means for administering the enzyme or composition to a subject. The kit may also further, or alternatively, comprise the gene therapy agent or a composition comprising it as described above. The kit may also comprise means for delivering the gene therapy agent. The kit may include instructions for use of the various components in any method as described herein.

### Further preferred embodiments

The following embodiments represent further preferred embodiments, but do not presently form claims, but may do so in the future.
[1] A method of treating a subject in need of gene therapy for a disease comprising:
   a. administering to said subject an amount of an enzyme effective to degrade or digest and/or inhibit or reduce effector function of serum antibodies in the subject; and
   b. administering the gene therapy to said subject;

   wherein the reaction products resulting from the activity of the enzyme prolong the serum half-life of the gene therapy in the subject and/or prolong or increase the exposure of the subject to the gene therapy;
   wherein the gene therapy is administered at a dose which is lower than the minimally effective dose that would be administered to a subject in the absence of step a.; and
   wherein said subject has no detectable antibodies that specifically bind to the gene therapy, or has either a low titre of neutralising antibodies that specifically bind to the gene therapy (NAb) or a low titre of total antibodies that specifically bind to the gene therapy (TAb), optionally wherein the presence/titre of antibodies in the subject is measured in a serum sample obtained from the subject prior to step a.
[2] The method according to embodiments [1], wherein the subject has a NAb titre of <1:10 or a TAb titre of <1:100.
[3] The method according to embodiment [1] or [2], wherein:
   - step a. is conducted between about 4 and about 24 hours, preferably about 4 to 6 hours, prior to step b; and/or
   - step a. and/or step b. are performed two or more times; and/or
   - the subject is a human.
[4] The method according to any one of embodiments [1] to [3], wherein said dose of enzyme administered in step a. is sufficient to degrade or digest and/or inhibit or reduce effector function of all or substantially all IgG molecules present in the serum of the subject, optionally wherein the enzyme is an IgG cysteine protease or an IgG endoglycosidase.
[5] The method according to embodiment [4], wherein:
   (i) the IgG cysteine protease is from a Streptococcus bacterium, such as Streptococcus pyogenes Streptococcus equi or Streptococcus zooepidemicus, optionally wherein said enzyme is a IdeS, MAC2, SpeB, IdeZ or IgdE polypeptide, or
   (ii) the IgG endoglycosidase is from a Streptococcus bacterium, such as Streptococcus pyogenes, Streptococcus equi or Streptococcus zooepidemicus, or from Corynebacterium pseudotuberculosis, Enterococcus faecalis, or Elizabethkingia meningoseptica, optionally wherein said enzyme is a EndoS, CP40, EndoE, or EndoF2 polypeptide.
[6] The method according to embodiment [4] or [5], wherein:
   - said IgG cysteine protease is a polypeptide comprising or consisting of a sequence that is at least 80% identical to SEQ ID NO: 2, 4, 5 such as at least 85%, 90%, 95%, 99% or 100% identical, or wherein said IgG cysteine protease comprises or consists of the sequence of any one of SEQ ID NOs: 6 to 25 and 55 to 69, 91 or 92, optionally wherein said sequence includes an additional methionine at the N terminus and/or a histidine tag at the C terminus; or
   - said IgG endoglycosidase is a polypeptide comprising or consisting of a sequence that is at least 80% identical to SEQ ID NO: 90, such as at least 85%, 90%, 95%, 99% or 100% identical, optionally wherein said sequence includes an additional methionine at the N terminus and/or a histidine tag at the C terminus.
[7] The method according to any one of embodiments [1] to [6], wherein the enzyme is imlifidase and/or EndoS.
[8] The method according to any one of embodiments [1] to [7], wherein the dose of enzyme is between 0.25 mg/kg BW and 0.5 mg/kg BW, preferably between 0.3 mg/kg BW and 0.5 mg/kg BW.
[9] The method according to any one of embodiments [1] to [8], wherein the gene therapy comprises a viral vector, optionally a lentiviral vector, an adenoviral vector or an adeno-associated virus (AAV) vector.
[10] The method according to embodiment 9, wherein said viral vector is administered at 2 × 10¹¹ vector genomes (vg)/kg body weight (vg/kg), lower than 1 × 10¹⁰ vg/kg, lower than 1 × 10⁹ vg/kg, lower than 1 × 10⁸ vg/kg, or lower than 0.99 × 10⁷.
[11] The method according to embodiment [9] or [10] wherein:
   - the gene therapy comprises a lentiviral vector which comprises envelope proteins to which antibodies of the subject specifically bind; or
   - the gene therapy comprises a AAV vector which comprises capsid proteins to which antibodies of the subject specifically bind, optionally VP1, VP2 and/or VP3 capsid proteins.
[12] The method according to embodiment [11], wherein said AAV vector comprises VP1 , VP2 and/or VP3 capsid protein having at least 60%, 70%, 80%, 90%, 95% or 100% sequence identity to VP1, VP2 and/or VP3 capsid protein selected from the group consisting of AAV1 , AAV2, AAV3, AAV4, AAV5, AAV6, AAV7, AAV8, AAV9, AAV10, AAV3B, AAV-2i8, RhlO, Rh74, SEQ ID NO: 93 and SEQ ID NO:94, VP1 , VP2 and/or VP3 capsid proteins.
[13] The method according to any one of embodiments [1] to [12], wherein the disease is:
   (i) caused by a loss of function or activity of a protein, and the gene therapy comprises a heterologous polynucleotide that encodes a protein or peptide that when expressed in the subject provides or supplements a function or activity of the protein; or
   (ii) caused by a gain of function activity or expression of a protein, and the gene therapy comprises a heterologous polynucleotide that is transcribed into a nucleic acid that inhibits, decreases or reduces expression of the gain of function, activity or expression of said protein.
[14] The method according to any one of embodiments [1] to [13], wherein the disease is selected from the group consisting of proliferative diseases (cancers, tumors, dysplasias, etc.), Crigler-Najjar and metabolic diseases like metabolic diseases of the liver, Friedreich ataxia, infectious diseases, addiction (e.g ., to tobacco, alcohol, or drugs), epilepsy, Canavan's disease, adrenoleukodystrophy, viral diseases (induced, e.g., by hepatitis B or C viruses, HIV, herpes, retroviruses, etc.), genetic diseases (cystic fibrosis, dystroglycanopathies, myopathies such as Duchenne muscular myopathy or dystrophy, myotubular myopathy, hemophilia A, hemophilia B, sickle-cell anemia, sickle cell disease, Fanconi's anemia, diabetes, amyotrophic lateral sclerosis (AFS), myotubularin myopathy, motor neuron diseases such as spinal muscular atrophy (SMA), spinobulbar muscular atrophy, or Charcot-Marie-Tooth disease, arthritis, severe combined immunodeficiencies (such as RS- SCID, ADA-SCID or X-SCID), Wiskott-Aldrich syndrome, X-linked thrombocytopenia, X-linked congenital neutropenia, chronic granulomatous disease, etc.), clotting factor deficiencies, cardiovascular disease (restenosis, ischemia, dyslipidemia, homozygous familial hypercholesterolemia, etc.), eye diseases such as retinitis pigmentosa, Feber congenital amaurosis, Feber hereditary optic neuropathy, and Stargardt disease; lysosomal storage diseases such as San Filippo syndrome; hyperbilirubinemia such as CN type I or II or Gilbert's syndrome; Fabry disease, glycogen storage disease such as GSDI, GSDII (Pompe disease), GSDIII, GSDIV, GSDV, GSDVI, GSDVII, GSDVIII and lethal congenital glycogen storage disease of the heart; and optionally wherein the vector comprises a therapeutic polynucleotide appropriate for treating the disease.
[15] The method according to embodiment [13](i )or [14] wherein said subject has:
   - a lung disease ( e.g ., cystic fibrosis); a bleeding disorder (e.g., hemophilia A or hemophilia B with or without inhibitors), thalassemia, a blood disorder (e.g., anemia), Alzheimer's disease, Parkinson's disease, Huntington's disease, amyotrophic lateral sclerosis (ALS), epilepsy, a lysosomal storage disease, )e.g., aspartylglucosaminuria, Batten disease, late infantile neuronal ceroid lipofuscinosis type 2 (CLN2), cystinosis, Fabry disease, Gaucher disease types I, II, and III, glycogen storage disease II (Pompe disease), GM2-gangliosidosis type I (Tay Sachs disease), GM2-gangliosidosis type II (Sandhoff disease), mucolipidosis types I (sialidosis type I and II), II (I-cell disease), III (pseudo-Hurler disease) and IV, mucopolysaccharide storage diseases (Hurler disease and variants, Hunter, Sanfilippo Types A,B,C,D, Morquio Types A and B, Maroteaux-Lamy and Sly diseases), Niemann-Pick disease types A/B, Cl and C2, and Schindler disease types I and II), hereditary angioedema (HAE), a copper or iron accumulation disorder (e.g., Wilson's or Menkes disease), lysosomal acid lipase deficiency, a neurological or neurodegenerative disorder, cancer, type 1 or type 2 diabetes, adenosine deaminase deficiency, a metabolic defect (e.g., glycogen storage diseases), a disease of solid organs (e.g., brain, liver, kidney, heart), or an infectious viral (e.g., hepatitis B and C, HIV, etc.), bacterial or fungal disease;
   - a blood clotting disorder, optionally hemophilia A, hemophilia A with inhibitory antibodies, hemophilia B, hemophilia B with inhibitory antibodies, a deficiency in any coagulation Factor: VII, VIII, IX, X, XI, V, XII, II, von Willebrand factor, or a combined FV/FVIII deficiency, thalassemia, vitamin K epoxide reductase Cl deficiency or gamma-carboxylase deficiency;
   - anemia, bleeding associated with trauma, injury, thrombosis, thrombocytopenia, stroke, coagulopathy, disseminated intravascular coagulation (DIC); over-anticoagulation associated with heparin, low molecular weight heparin, pentasaccharide, warfarin, small molecule antithrombotics (i.e., FXa inhibitors), or a platelet disorder such as, Bernard Soulier syndrome, Glanzmann thrombasthenia, or storage pool deficiency
[16] The method according to any one of embodiments [1] to [12], embodiment [13](i), [14] or [15], wherein the heterologous polynucleotide encodes:
   - a protein selected from the group consisting of insulin, glucagon, growth hormone (GH), parathyroid hormone (PTH), growth hormone releasing factor (GRF), follicle stimulating hormone (FSH), luteinizing hormone (LH), human chorionic gonadotropin (hCG), vascular endothelial growth factor (VEGF), angiopoietins, angiostatin, granulocyte colony stimulating factor (GCSF), erythropoietin (EPO), connective tissue growth factor (CTGF), basic fibroblast growth factor (bFGF), acidic fibroblast growth factor (aFGF), epidermal growth factor (EGF), transforming growth factor a (TGFa), platelet-derived growth factor (PDGF), insulin growth factors I and II (IGF-I and IGF-II), TGF , activins, inhibins, bone morphogenic protein (BMP), nerve growth factor (NGF), brain-derived neurotrophic factor (BDNF), neurotrophins NT-3 and NT4/5, ciliary neurotrophic factor (CNTF), glial cell line derived neurotrophic factor (GDNF), neurturin, agrin, netrin-1 and netrin-2, hepatocyte growth factor (HGF), ephrins, noggin, sonic hedgehog and tyrosine hydroxylase; or
   - a protein selected from the group consisting of thrombopoietin (TPO), interleukins (IL-1 through IL-36), monocyte chemoattractant protein, leukemia inhibitory factor, granulocyte-macrophage colony stimulating factor, Fas ligand, tumor necrosis factors a and b, interferons a, b, and g, stem cell factor, flk-2/flt3 ligand, IgG, IgM, IgA, IgD and IgE, chimeric immunoglobulins, humanized antibodies, single chain antibodies, T cell receptors, chimeric T cell receptors, single chain T cell receptors, class I and class II MHC molecules; or
   - CFTR (cystic fibrosis transmembrane regulator protein), a blood coagulation (clotting) factor (Factor XIII, Factor IX, Factor VIII, Factor X, Factor VII, Factor Vila, protein C, etc.) a gain of function blood coagulation factor, an antibody, retinal pigment epithelium-specific 65 kDa protein (RPE65), erythropoietin, FDF receptor, lipoprotein lipase, ornithine transcarbamylase, b-globin, a-globin, spectrin, a-antitrypsin, adenosine deaminase (ADA), a metal transporter (ATP7A or ATP7), sulfamidase, an enzyme involved in lysosomal storage disease (ARSA), hypoxanthine guanine phosphoribosyl transferase, b-25 glucocerebrosidase, sphingomyelinase, lysosomal hexosaminidase, branched-chain keto acid dehydrogenase, a hormone, a growth factor, insulin-like growth factor 1 or 2, platelet derived growth factor, epidermal growth factor, nerve growth factor, neurotrophic factor -3 and -4, brain-derived neurotrophic factor, glial derived growth factor, transforming growth factor a and b, a cytokine, a-interferon, b-interferon, interferon-g, interleukin-2, interleukin-4, interleukin 12, granulocyte-macrophage colony stimulating factor, lymphotoxin, a suicide gene product, herpes simplex virus thymidine kinase, cytosine deaminase, diphtheria toxin, cytochrome P450, deoxycytidine kinase, tumor necrosis factor, a drug resistance protein, a tumor suppressor protein ( e.g ., p53, Rb, Wt-l , NF1, Von Hippel-Findau (VHF), adenomatous polyposis coli (APC)), a peptide with immunomodulatory properties, a tolerogenic or immunogenic peptide or protein Tregitope or hCDRl, insulin, glucokinase, guanylate cyclase 2D (FCA-GUCY2D), Rab escort protein 1 (Choroideremia), FCA 5 (FCA-Febercilin), ornithine ketoacid aminotransferase (Gyrate Atrophy), Retinoschisin 1 (X-linked Retinoschisis), USH1C (Usher's Syndrome 1C), X-linked retinitis pigmentosa GTPase (XFRP), MERTK (AR forms of RP: retinitis pigmentosa), DFNB1 (Connexin 26 deafness), ACHM 2, 3 and 4 (Achromatopsia), PKD-1 or PKD-2 (Polycystic kidney disease), TPP1, CFN2, a sulfatase, N-acetylglucosamine-l -phosphate transferase, cathepsin A, GM2-AP, NPC1, VPC2, a sphingolipid activator protein, one or more zinc finger nuclease for genome editing, and one or more donor sequence used as repair templates for genome editing; or
   - a gene editing nuclease, which optionally comprises a zinc finger nuclease (ZFN), a transcription activator-like effector nuclease (TALEN), or a functional Type II CRISPR-Cas9.
[17] The method according to any one of embodiments [1] to [12] and embodiment [13](ii) or [14], wherein said inhibitory nucleic acid binds to a gene, a transcript of a gene, or a transcript of a gene associated with a polynucleotide repeat disease selected from the group consisting of a huntingtin (HTT) gene, a gene associated with dentatorubropallidoluysian atrophy (atrophin 1, ATN1), androgen receptor on the X chromosome in spinobulbar muscular atrophy, human Ataxin-1, -2, -3, and -7, Ca v 2.1 P/Q voltage-dependent calcium channel (CACNA1A), TATA-binding protein, Ataxin 8 opposite strand (ATXN80S), Serine/threonine-protein phosphatase 2A 55 kDa regulatory subunit B beta isoform in spinocerebellar ataxia (type 1, 2, 3, 6, 7, 8, 12 17), FMR1 (fragile X mental retardation 1) in fragile X syndrome, FMR1 (fragile X mental retardation 1) in fragile X-associated tremor/ataxia syndrome, FMR1 (fragile X mental retardation 2) or AF4/FMR2 family member 2 in fragile XE mental retardation; Myotonin-protein kinase (MT-PK) in myotonic dystrophy; Frataxin in Friedreich's ataxia; a mutant of superoxide dismutase 1 (SOD1) gene in amyotrophic lateral sclerosis; a gene involved in pathogenesis of Parkinson's disease and/or Alzheimer's disease; apolipoprotein B (APOB) and proprotein convertase subtilisin/kexin type 9 (PCSK9), hypercholesterolemia; HIV Tat, human immunodeficiency virus transactivator of transcription gene, in HIV infection; HIV TAR, HIV TAR, human immunodeficiency virus transactivator response element gene, in HIV infection; C-C chemokine receptor (CCR5) in HIV infection; Rous sarcoma virus (RSV) nucleocapsid protein in RSV infection, liver-specific microRNA (miR-122) in hepatitis C virus infection; p53, acute kidney injury or delayed graft function kidney transplant or kidney injury acute renal failure; protein kinase N3 (PKN3) in advance recurrent or metastatic solid malignancies; LMP2, LMP2 also known as proteasome subunit beta-type 9 (PSMB 9), metastatic melanoma; LMP7,also known as proteasome subunit beta-type 8 (PSMB 8), metastatic melanoma; MECL1 also known as proteasome subunit beta-type 10 (PSMB 10), metastatic melanoma; vascular endothelial growth factor (VEGF) in solid tumors; kinesin spindle protein in solid tumors, apoptosis suppressor B- cell CLL/lymphoma (BCL-2) in chronic myeloid leukemia; ribonucleotide reductase M2 (RRM2) in solid tumors; Furin in solid tumors; polo-like kinase 1 (PLK1) in liver tumors, diacylglycerol acyltransferase 1 (DGAT1) in hepatitis C infection, beta-catenin in familial adenomatous polyposis; beta2 adrenergic receptor, glaucoma; RTP801/Reddl also known as DNA damage-inducible transcript 4 protein, in diabetic macular edema (DME) or age- related macular degeneration; vascular endothelial growth factor receptor I (VEGFR1) in age-related macular degeneration or choroidal neovascularization, caspase 2 in non- arteritic ischaemic optic neuropathy; Keratin 6 A N 17K mutant protein in pachyonychia congenital; influenza A virus genome/gene sequences in influenza infection; severe acute respiratory syndrome (SARS) coronavirus genome/gene sequences in SARS infection; respiratory syncytial virus genome/gene sequences in respiratory syncytial virus infection; Ebola filovirus genome/gene sequence in Ebola infection; hepatitis B and C virus genome/gene sequences in hepatitis B and C infection; herpes simplex virus (HSV) genome/gene sequences in HSV infection, coxsackievirus B3 genome/gene sequences in coxsackievirus B3 infection; silencing of a pathogenic allele of a gene (allele-specific silencing) like torsin A (TORI A) in primary dystonia, pan-class I and HLA-allele specific in transplant; and mutant rhodopsin gene (RHO) in autosomal dominantly inherited retinitis pigmentosa (adRP); and wherein said inhibitory nucleic acid is optionally a siRNA, an antisense molecule, miRNA, RNAi, a ribozyme or a shRNA.

As well as embodiments [1] to [17] outlined above, the present invention also provides an enzyme as described herein for use in any of the methods set out in embodiments [1] to [17]. The present invention further provides a gene therapy agent as described herein for any of the methods set out in embodiments [1] to [17].

### EXAMPLES

Unless indicated otherwise, the methods used are standard biochemistry and molecular biology techniques. Examples of suitable methodology textbooks include Sambrook et al., Molecular Cloning, A Laboratory Manual (1989) and Ausubel et al., Current Protocols in Molecular Biology (1995), John Wiley and Sons, Inc.

### Example 1 - optimal spacing of imlifidase and rATG

### Materials and methods

### SCID mice experimental set up and sample collection

Immunocompromised CB17-SCID mice (Janvier Labs) were selected to evaluate the effect of imlifidase on the transduction and AAV kinetics in vivo. SCID mice are unable to produce antibodies to common antigens and their progenitor B and T-cells cannot differentiate because of impaired VDJ rearrangement. However, SCID mice contain relatively normal numbers of nonlymphoid cells like NK cells, macrophages and granulocytes. The SCID model was employed to exclude any contaminating mouse antibodies, which in contrast to human antibodies are not efficiently cleaved by imlifidase. A pool of IgG antibodies (IVIg, Privigen, Batch# P100322084), was used for the introduction of human IgG into these mice.

Female CB17-SCID mice (8 weeks old) were administered intraperitoneally (i.p.) with 200µL DPBS or intact IVIg (80mg/mL). One hour later the mice were given a 100µl intravenous (i.v.) injection of imlifidase or DPBS at a concentration of 1mg/mL (Baxter, batch#0L008A). Four hours later the mice were given an i.v. injection of 100µl AAV viral vector (AAV8-CMV-LUC at 1×10¹² vg/ml) or DPBS, hence the AAV viral vector was given 5 hours after the original IVIg injection. Throughout the study, blood samples were collected from sublingual vein at defined time-points relative to the AAV injection. At day 14, the mice were sacrificed and the terminal blood sample was collected by heart puncture under anaesthetic conditions with isoflurane. Tissue samples (heart and liver) were dissected, weighed and snap frozen in isopentane for later analysis of AAV levels by qPCR.

### Quantification of the viral genome

At the end of the study, transduction capacity of the virus and thereby the ability to reach the tissue target was evaluated by qPCR (quantitative polymerase chain reaction). The viral vector targets the liver but this is also considered an organ where virus is cleared from the circulation by Kupffer cells. The results seen in the liver were therefore further confirmed using another target tissue, the heart. The presence and clearance of the virus in the blood after i.v. injection was followed in the plasma throughout the study and quantified by qPCR.

### qPCR in plasma

To quantify the numbers of AAV vector genomes in the circulation of the mice at a given time-point after administration, qPCR was applied. At predefined time-points post AAV administration (1, 4, 24, 48, 168, 336 hours), blood was collected in Li-Hep coated vials, centrifuged at 2000xg for 5 minutes at 4°C. Plasma was retrieved and transferred to 1.5-mL Eppendorf tubes, frozen on dry ice and kept in -80°C freezer until analysis.
Plasma samples from individual mice were diluted 1:20 in nuclease free water in a 96-well plate and thereafter incubated in a heat block for 10min at 70°C to release viral DNA from the capsids. Subsequently, samples were analyzed with qPCR detecting a viral specific gene (ITR). In each well of a 96-well plate as 10µL 2x SSoAdvanced Universal probe supermix (Biorad, Cat#1725281, 1µL TaqMan assay (primer sequence supplied separately), 2µL template and 7µL nuclease free water were added. Each sample was analysed in triplicate. The 96 well plate was covered with optical seal and run on AriaMx Real time PCR system according to the following reaction conditions; 95°C for 5 minutes, followed by 45 cycles at 95°C for 15 seconds, 60°C for 15 seconds and 72°C for 15 seconds. The results obtained were also further confirmed by qPCR using another primer targeting the luciferase gene (data not shown).

With the use of a standard curve (linearized AAV vector at known copies/µL concentrations) the Cq values received in qPCR were translated into viral genome copies/µL plasma.

### qPCR in liver lysate

To quantify the numbers of AAV vector genomes that successfully enter the liver target tissue after viral administration, quantitative PCR (qPCR) was applied.
At termination (Day 14) the whole liver was dissected out and weighed. One selected liver lobe was separated, weighed for confirmation, and placed in a 2mL Eppendorf tube containing 700µL of 1x Cell culture lysis reagent (Promega, Cat#E1531) and 6.35-mm ceramic sphere beads (MP Biomedicals, Cat#6540-412. The samples were homogenized using a Bead Mill 4 device (Fisherbrand) at 3x30 seconds with a speed of 5m/s and 15 second pauses between the cycles. The vials were centrifuged at 20,000xg for 2 minutes at 4°C and supernatants transferred to 1.5mL tubes in two aliquots and stored at -80°C until used. The remaining liver was divided in 2 parts, weighed and snap frozen in isopentane and stored at - 80°C.

Total DNA (genomic, mitochondrial and viral) was extracted from the liver lysate using DNeasy blood and tissue kit (Qiagen, Cat#69504) according to protocol supplied by the manufacturer. In brief, a fraction of the lysate was used so that the capacity of the DNA binding column is not exceeded. The lysate was applied to the column with silica based membrane which selectively bind DNA and bound DNA was thereafter washed and eluted in 10mM Tris-HCl with 0.5mM EDTA, pH9.

Extracted total DNA was diluted 25 times in sterile water and then used as template in a qPCR reaction using two sets of primers, specific either for the viral genome or a genomic reference gene, to be able to calculate relative viral content per liver cell. To each well of a 96-well plate 10µl 2x SSoAdvanced Universal probe supermix (Biorad, Cat#1725281), 1µl TaqMan assay (20x, primer sequence supplied separately), 5µl DNA template and 4µl nuclease free water was added. Each sample was analyzed in triplicate and with one set of triplicate wells including primers detecting the viral specific gene (ITR) and one set with the reference gene (Actin). The 96 well plate was covered with optical seal and run on AriaMx Real time PCR system according to the following reaction conditions; 95°C for 3 minutes, 40 cycles at 95°C for 15 seconds and 60°C for 30 seconds, detecting FAM signals and using ROX as passive reference.

The viral gene Cq values received were normalized to the reference gene, which presence is constant in the mouse genome, thus generating a relative AAV genome content/cell that could be used for comparisons between groups. As calibrator to remove any background signal, non-treated AAV samples (only injected with IVIg and imlifidase) were used.

### qPCR in heart

To quantify the numbers of AAV vector genomes that successfully entered the heart target tissue after viral administration, qPCR was employed. At termination (Day 14) heart tissue samples was collected, under anaesthetic condition with isoflurane, weighed and snap frozen in isopentane. Total DNA (genomic, mitochondrial and viral) was extracted from the heart tissue using DNeasy Blood and tissue kit (Qiagen), according to protocol supplied by the manufacturer. In brief, a fraction of the heart tissue was excised, weighed and thereafter sliced into smaller pieces before lysed in buffer containing proteinase K at 56°C for 4 hours. The lysate was applied to a column with silica based membrane which selectively bind DNA. Bound DNA was thereafter washed and eluted in 10mM Tris-HCl with 0.5mM EDTA, pH9. Extracted total DNA was diluted 25 times in sterile water and then used as template in qPCR reaction using two sets of primers binding either the viral or a reference gene to be able to calculate relative viral content per heart cell.

To each well of a 96-well plate 10µl 2x SSoAdvanced Universal probe supermix (Biorad, Cat# 1725281), 1µl TaqMan assay (20x, primer sequence supplied separately), 5µl DNA template and 4µl nuclease free water was added. Each sample was analyzed in triplicate and for each sample both primers detecting the viral specific gene (ITR) and the reference gene (Actin or GAPDH) were separately used for amplification. The 96 well plate was covered with optical seal and run on AriaMx Real time PCR system according to the following reaction conditions; 95°C for 3 minutes, 40 cycles at 95°C for 15 seconds and 60°C for 30 seconds, detecting FAM signals and using ROX as passive reference. The viral gene Cq values received were normalized to the reference gene, which presence is constant in the mice genome, thus generating a relative AAV content/cell that could be used for comparisons between groups. As calibrator to remove any background signal, non-treated AAV samples (only injected with IVIg and imlifidase) were used. The primers employed are given in Table 1 below.

**Table 1 : Primer sequences**

| TaqMan assay | Target | Assay ID | Primer (5'-3') sequence |
|---|---|---|---|
| Off the shelf | Actin | Mm01205647_g1 | Non disclosed by Thermo Fisher |
| | | | Scientific |
| Off the shelf | GAPDH | Mm00186825_cn | Non disclosed by Thermo Fisher |
| | | | Scientific |
| | | | Forward: |
| | | | GGAACCCCTAGTGATGGAGTT |
| | | | Reverse: CGGCCTCAGTGAGCGA |
| | | | Probe: |
| Custom made | ITR | APNKXMW | CACTCCCTCTCTGCGCGCTCG |

### Results

The results obtained allowed a comparison of AAV levels up to 14 days after administration of the AAV gene therapy vector as measured by qPCR. The experiments compared the following three groups:
- SCID mice given human intravenous antibodies (IVIg), followed subsequently by AAV;
- SCID mice given human intravenous antibodies (IVIg) and Ides, followed subsequently by AAV; and
- SCID mice only given AAV at the same time-point as the other mice.

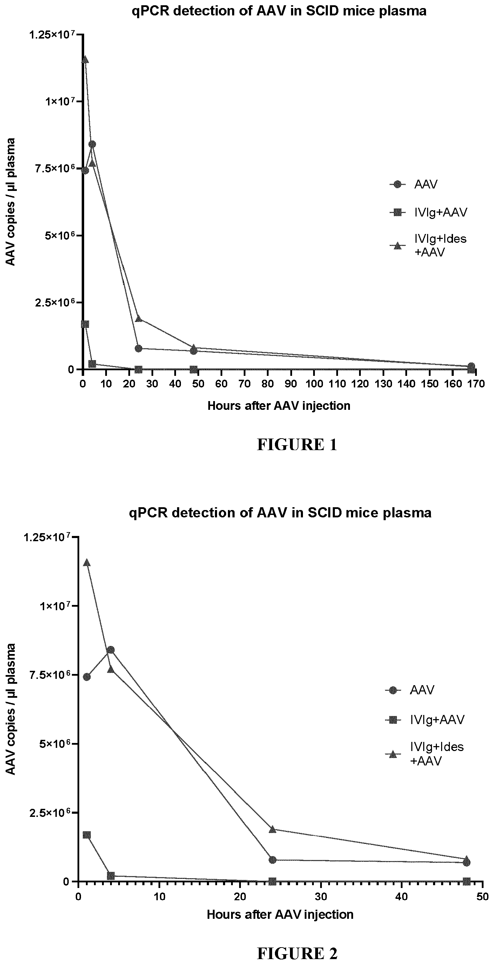

The results obtained are shown in Figures 1 to 5.

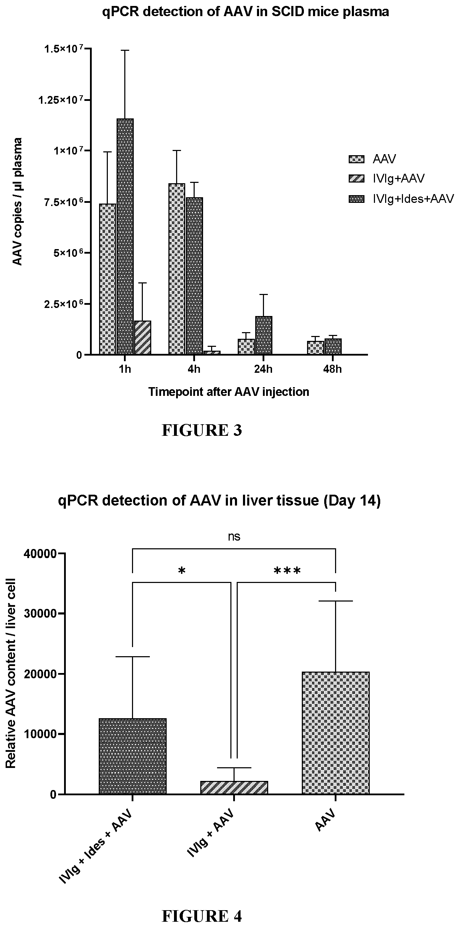

Figure 1 shows the level of AAV in serum plasma up to 7 days after the administration of the AAV in the three groups. Figure 1 shows that in the group where IVIg and AAV were both given (squares), the AAV was quickly cleared and already after 4 hours the level of AAVs in plasma was close to the detection limit. In the group administered AAV alone (circles) the level of AAV was more slowly reduced and still detectable at Day 7 (168 hours). In the group of mice where IVIg + Ides were injected prior to AAV administration (triangles) the kinetics of the AAV levels were similar or even improved compared to AAV alone, with a slower pace for clearance and improved peak levels. Figure 2 shows results from the same experiment as Figure 1, but for serum plasma AAV levels over only the first 48 hours of the experiment. Again, the graph shows that the IdeS appears to give a benefit even in the absence of administration of IVIg. Figure 3 also shows results from the same experiment, again with serum AAV levels, but this time with the results depicted in a bar chart showing the result at 1 hour, 4 hours, 24 hours, and 48 hours after AAV administration. Again, the results show that administration of Ides negates the effect of the IVIg and even in the absence of AAV appears to boost AAV levels at several of the time-points measured.

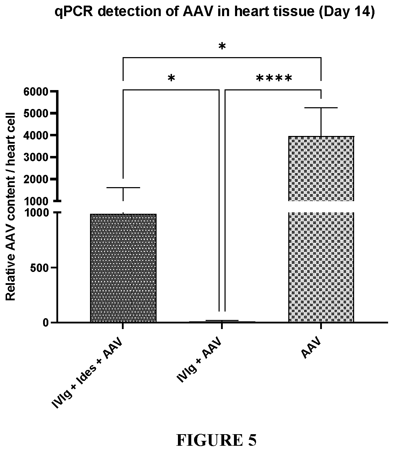

Figure 4 shows AAV levels in the liver at the end of the experiment 14 days after AAV administration. The group administered AAV and IVIg had almost cleared the AAV after 14 days. In contrast, the group administered AAV, IVIg and Ides did not have a significantly different AAV level in the liver after 14 days compared to the group administered AAV alone. Figure 5 shows AAV levels in the heart 14 days after AAV administration. Again, the group given IVIG and AAV had almost completely cleared the AAV. Whilst the group given AAV alone did have significantly higher AAV levels in the heart at 14 days in comparison to the group given IVIg, Ides and AAV, the latter group still had far higher AAV levels persisting in comparison to the IVIg plus AAV group.

Thus, surprisingly, the rate of AAV clearance from serum plasma of the SCID mice when Ides was present was even better than that seen in the group administered AAV without any IVIg. The administration of the Ides also dramatically slowed clearance of the AAV from the liver and heart at 14 days, the end of the experiment. The results therefore illustrates that enzymes such as Ides may be used to negate the impact of antibodies on gene therapy, but that it may be of benefit even in the absence of antibodies targeting the gene therapy vector.

## Claims

1. An enzyme for use in a method of treating a subject in need of gene therapy for a disease comprising:
a. administering to said subject an amount of the enzyme effective to degrade or digest and/or inhibit or reduce effector function of serum antibodies in the subject; and
b. administering the gene therapy to said subject;
wherein the reaction products resulting from the activity of the enzyme prolong the serum half-life of the gene therapy in the subject and/or prolong or increase the exposure of the subject to the gene therapy;
wherein the gene therapy is administered at a dose which is lower than the minimally effective dose that would be administered to a subject in the absence of step a.; and
wherein said subject has no detectable antibodies that specifically bind to the gene therapy, or has either a low titre of neutralising antibodies that specifically bind to the gene therapy (NAb) or a low titre of total antibodies that specifically bind to the gene therapy (TAb), optionally wherein the presence/titre of antibodies in the subject is measured in a serum sample obtained from the subject prior to step a.

2. The enzyme for use according to claim 1, wherein the subject has a NAb titre of <1:10 or a TAb titre of <1:100.

3. The enzyme for use according to claim 1 or 2, wherein:
- step a. is conducted between about 4 and about 72 hours, for instance about 4 hours to about 24 hours, preferably about 4 to 6 hours, prior to step b; and/or
- step a. and/or step b. are performed two or more times; and/or
- the subject is a human.

4. The enzyme for use according to any one of the preceding claims wherein said dose of enzyme administered in step a. is sufficient to degrade or digest and/or inhibit or reduce effector function of all or substantially all IgG molecules present in the serum of the subject, optionally wherein the enzyme is an IgG cysteine protease or an IgG endoglycosidase.

5. The enzyme for use according to claim 4, wherein:
(i) the IgG cysteine protease is from a Streptococcus bacterium, such as Streptococcus pyogenes Streptococcus equi or Streptococcus zooepidemicus, optionally wherein said enzyme is a IdeS, MAC2, SpeB, IdeZ or IgdE polypeptide, or
(ii) the IgG endoglycosidase is from a Streptococcus bacterium, such as Streptococcus pyogenes, Streptococcus equi or Streptococcus zooepidemicus, or from Corynebacterium pseudotuberculosis, Enterococcus faecalis, or Elizabethkingia meningoseptica, optionally wherein said enzyme is a EndoS, CP40, EndoE, or EndoF2 polypeptide.

6. The enzyme for use according to claim 5 or 6, wherein:
- said IgG cysteine protease is a polypeptide comprising or consisting of a sequence that is at least 80% identical to SEQ ID NO: 2, 4, 5 such as at least 85%, 90%, 95%, 99% or 100% identical, or wherein said IgG cysteine protease comprises or consists of the sequence of any one of SEQ ID NOs: 6 to 25 and 55 to 69, 91 or 92, optionally wherein said sequence includes an additional methionine at the N terminus and/or a histidine tag at the C terminus; or
- said IgG endoglycosidase is a polypeptide comprising or consisting of a sequence that is at least 80% identical to SEQ ID NO: 90, such as at least 85%, 90%, 95%, 99% or 100% identical, optionally wherein said sequence includes an additional methionine at the N terminus and/or a histidine tag at the C terminus.

7. The enzyme for use according to any one of the preceding claims wherein the enzyme is imlifidase and/or EndoS.

8. The enzyme for use according to any one of the preceding claims wherein the dose of enzyme is between 0.25 mg/kg BW and 0.5 mg/kg BW, preferably between 0.3 mg/kg BW and 0.5 mg/kg BW.

9. The enzyme for use according to any one of the preceding claims, wherein the gene therapy comprises a viral vector, optionally a lentiviral vector, an adenoviral vector or an adeno-associated virus (AAV) vector.

10. The enzyme for use according to claim 9, wherein said viral vector is administered at a dose which is lower than 2 × 10¹¹ vector genomes (vg)/kg body weight (vg/kg), lower than 1 × 10¹⁰ vg/kg, lower than 1 × 10⁹ vg/kg, lower than 1 × 10⁸ vg/kg, or lower than 0.99 × 10⁷.

11. The enzyme for use according to claim 9 or 10 wherein:
- the gene therapy comprises a lentiviral vector which comprises envelope proteins to which antibodies of the subject specifically bind; or
- the gene therapy comprises a AAV vector which comprises capsid proteins to which antibodies of the subject specifically bind, optionally VP1, VP2 and/or VP3 capsid proteins.

12. The enzyme for use according to claim 11, wherein said AAV vector comprises VP1, VP2 and/or VP3 capsid protein having at least 60%, 70%, 80%, 90%, 95% or 100% sequence identity to VP1, VP2 and/or VP3 capsid protein selected from the group consisting of AAV1, AAV2, AAV3, AAV4, AAV5, AAV6, AAV7, AAV8, AAV9, AAV10, AAV3B, AAV-2i8, RhlO, Rh74, SEQ ID NO: 93 and SEQ ID NO:94, VP1 , VP2 and/or VP3 capsid proteins.

13. The enzyme for use according to any one of the preceding claims, wherein the disease is:
(i) caused by a loss of function or activity of a protein, and the gene therapy comprises a heterologous polynucleotide that encodes a protein or peptide that when expressed in the subject provides or supplements a function or activity of the protein; or
(ii) caused by a gain of function activity or expression of a protein, and the gene therapy comprises a heterologous polynucleotide that is transcribed into a nucleic acid that inhibits, decreases or reduces expression of the gain of function, activity or expression of said protein.

14. The enzyme for use according to any one of the preceding claims, wherein the disease is selected from the group consisting of proliferative diseases (cancers, tumors, dysplasias, etc.), Crigler-Najjar and metabolic diseases like metabolic diseases of the liver, Friedreich ataxia, infectious diseases, addiction (e.g ., to tobacco, alcohol, or drugs), epilepsy, Canavan's disease, adrenoleukodystrophy, viral diseases (induced, e.g., by hepatitis B or C viruses, HIV, herpes, retroviruses, etc.), genetic diseases (cystic fibrosis, dystroglycanopathies, myopathies such as Duchenne muscular myopathy or dystrophy, myotubular myopathy, hemophilia A, hemophilia B, sickle-cell anemia, sickle cell disease, Fanconi's anemia, diabetes, amyotrophic lateral sclerosis (AFS), myotubularin myopathy, motor neuron diseases such as spinal muscular atrophy (SMA), spinobulbar muscular atrophy, or Charcot-Marie-Tooth disease, arthritis, severe combined immunodeficiencies (such as RS- SCID, ADA-SCID or X-SCID), Wiskott-Aldrich syndrome, X-linked thrombocytopenia, X-linked congenital neutropenia, chronic granulomatous disease, etc.), clotting factor deficiencies, cardiovascular disease (restenosis, ischemia, dyslipidemia, homozygous familial hypercholesterolemia, etc.), eye diseases such as retinitis pigmentosa, Feber congenital amaurosis, Feber hereditary optic neuropathy, and Stargardt disease; lysosomal storage diseases such as San Filippo syndrome; hyperbilirubinemia such as CN type I or II or Gilbert's syndrome; Fabry disease, glycogen storage disease such as GSDI, GSDII (Pompe disease), GSDIII, GSDIV, GSDV, GSDVI, GSDVII, GSDVIII and lethal congenital glycogen storage disease of the heart; and optionally wherein the vector comprises a therapeutic polynucleotide appropriate for treating the disease.

15. The enzyme for use according to claim 13(i) or 14 wherein said subject has:
- a lung disease ( e.g ., cystic fibrosis); a bleeding disorder (e.g., hemophilia A or hemophilia B with or without inhibitors), thalassemia, a blood disorder (e.g., anemia), Alzheimer's disease, Parkinson's disease, Huntington's disease, amyotrophic lateral sclerosis (ALS), epilepsy, a lysosomal storage disease, )e.g., aspartylglucosaminuria, Batten disease, late infantile neuronal ceroid lipofuscinosis type 2 (CLN2), cystinosis, Fabry disease, Gaucher disease types I, II, and III, glycogen storage disease II (Pompe disease), GM2-gangliosidosis type I (Tay Sachs disease), GM2-gangliosidosis type II (Sandhoff disease), mucolipidosis types I (sialidosis type I and II), II (I-cell disease), III (pseudo-Hurler disease) and IV, mucopolysaccharide storage diseases (Hurler disease and variants, Hunter, Sanfilippo Types A,B,C,D, Morquio Types A and B, Maroteaux-Lamy and Sly diseases), Niemann-Pick disease types A/B, Cl and C2, and Schindler disease types I and II), hereditary angioedema (HAE), a copper or iron accumulation disorder (e.g., Wilson's or Menkes disease), lysosomal acid lipase deficiency, a neurological or neurodegenerative disorder, cancer, type 1 or type 2 diabetes, adenosine deaminase deficiency, a metabolic defect (e.g., glycogen storage diseases), a disease of solid organs (e.g., brain, liver, kidney, heart), or an infectious viral (e.g., hepatitis B and C, HIV, etc.), bacterial or fungal disease;
- a blood clotting disorder, optionally hemophilia A, hemophilia A with inhibitory antibodies, hemophilia B, hemophilia B with inhibitory antibodies, a deficiency in any coagulation Factor: VII, VIII, IX, X, XI, V, XII, II, von Willebrand factor, or a combined FV/FVIII deficiency, thalassemia, vitamin K epoxide reductase Cl deficiency or gamma-carboxylase deficiency;
- anemia, bleeding associated with trauma, injury, thrombosis, thrombocytopenia, stroke, coagulopathy, disseminated intravascular coagulation (DIC); over-anticoagulation associated with heparin, low molecular weight heparin, pentasaccharide, warfarin, small molecule antithrombotics (i.e., FXa inhibitors), or a platelet disorder such as, Bernard Soulier syndrome, Glanzmann thrombasthenia, or storage pool deficiency

16. The enzyme for use according to any one of claims 1 to 12, claim 13(i), 14 or 15, wherein the heterologous polynucleotide encodes:
- a protein selected from the group consisting of insulin, glucagon, growth hormone (GH), parathyroid hormone (PTH), growth hormone releasing factor (GRF), follicle stimulating hormone (FSH), luteinizing hormone (LH), human chorionic gonadotropin (hCG), vascular endothelial growth factor (VEGF), angiopoietins, angiostatin, granulocyte colony stimulating factor (GCSF), erythropoietin (EPO), connective tissue growth factor (CTGF), basic fibroblast growth factor (bFGF), acidic fibroblast growth factor (aFGF), epidermal growth factor (EGF), transforming growth factor a (TGFa), platelet-derived growth factor (PDGF), insulin growth factors I and II (IGF-I and IGF-II), TGF , activins, inhibins, bone morphogenic protein (BMP), nerve growth factor (NGF), brain-derived neurotrophic factor (BDNF), neurotrophins NT-3 and NT4/5, ciliary neurotrophic factor (CNTF), glial cell line derived neurotrophic factor (GDNF), neurturin, agrin, netrin-l and netrin-2, hepatocyte growth factor (HGF), ephrins, noggin, sonic hedgehog and tyrosine hydroxylase; or
- a protein selected from the group consisting of thrombopoietin (TPO), interleukins (IL-1 through IL-36), monocyte chemoattractant protein, leukemia inhibitory factor, granulocyte-macrophage colony stimulating factor, Fas ligand, tumor necrosis factors a and b, interferons a, b, and g, stem cell factor, flk-2/flt3 ligand, IgG, IgM, IgA, IgD and IgE, chimeric immunoglobulins, humanized antibodies, single chain antibodies, T cell receptors, chimeric T cell receptors, single chain T cell receptors, class I and class II MHC molecules; or
- CFTR (cystic fibrosis transmembrane regulator protein), a blood coagulation (clotting) factor (Factor XIII, Factor IX, Factor VIII, Factor X, Factor VII, Factor Vila, protein C, etc.) a gain of function blood coagulation factor, an antibody, retinal pigment epithelium-specific 65 kDa protein (RPE65), erythropoietin, FDF receptor, lipoprotein lipase, ornithine transcarbamylase, b-globin, a-globin, spectrin, a-antitrypsin, adenosine deaminase (ADA), a metal transporter (ATP7A or ATP7), sulfamidase, an enzyme involved in lysosomal storage disease (ARSA), hypoxanthine guanine phosphoribosyl transferase, b-25 glucocerebrosidase, sphingomyelinase, lysosomal hexosaminidase, branched-chain keto acid dehydrogenase, a hormone, a growth factor, insulin-like growth factor 1 or 2, platelet derived growth factor, epidermal growth factor, nerve growth factor, neurotrophic factor -3 and -4, brain-derived neurotrophic factor, glial derived growth factor, transforming growth factor a and b, a cytokine, a-interferon, b-interferon, interferon-g, interleukin-2, interleukin-4, interleukin 12, granulocyte-macrophage colony stimulating factor, lymphotoxin, a suicide gene product, herpes simplex virus thymidine kinase, cytosine deaminase, diphtheria toxin, cytochrome P450, deoxycytidine kinase, tumor necrosis factor, a drug resistance protein, a tumor suppressor protein ( e.g ., p53, Rb, Wt-l, NF1, Von Hippel-Findau (VHF), adenomatous polyposis coli (APC)), a peptide with immunomodulatory properties, a tolerogenic or immunogenic peptide or protein Tregitope or hCDRl, insulin, glucokinase, guanylate cyclase 2D (FCA-GUCY2D), Rab escort protein 1 (Choroideremia), FCA 5 (FCA-Febercilin), ornithine ketoacid aminotransferase (Gyrate Atrophy), Retinoschisin 1 (X-linked Retinoschisis), USH1C (Usher's Syndrome 1C), X-linked retinitis pigmentosa GTPase (XFRP), MERTK (AR forms of RP: retinitis pigmentosa), DFNB1 (Connexin 26 deafness), ACHM 2, 3 and 4 (Achromatopsia), PKD-1 or PKD-2 (Polycystic kidney disease), TPP1, CFN2, a sulfatase, N-acetylglucosamine-l -phosphate transferase, cathepsin A, GM2-AP, NPC1, VPC2, a sphingolipid activator protein, one or more zinc finger nuclease for genome editing, and one or more donor sequence used as repair templates for genome editing; or
- a gene editing nuclease, which optionally comprises a zinc finger nuclease (ZFN), a transcription activator-like effector nuclease (TALEN), or a functional Type II CRISPR-Cas9.

17. The enzyme for use according to any one of claims 1 to 12 and claim 13(ii) or 14, wherein said inhibitory nucleic acid binds to a gene, a transcript of a gene, or a transcript of a gene associated with a polynucleotide repeat disease selected from the group consisting of a huntingtin (HTT) gene, a gene associated with dentatorubropallidoluysian atrophy (atrophin 1, ATN1), androgen receptor on the X chromosome in spinobulbar muscular atrophy, human Ataxin-1, -2, -3, and -7, Ca v 2.1 P/Q voltage-dependent calcium channel (CACNA1A), TATA-binding protein, Ataxin 8 opposite strand (ATXN80S), Serine/threonine-protein phosphatase 2A 55 kDa regulatory subunit B beta isoform in spinocerebellar ataxia (type 1, 2, 3, 6, 7, 8, 12 17), FMR1 (fragile X mental retardation 1) in fragile X syndrome, FMR1 (fragile X mental retardation 1) in fragile X-associated tremor/ataxia syndrome, FMR1 (fragile X mental retardation 2) or AF4/FMR2 family member 2 in fragile XE mental retardation; Myotonin-protein kinase (MT-PK) in myotonic dystrophy; Frataxin in Friedreich's ataxia; a mutant of superoxide dismutase 1 (SOD1) gene in amyotrophic lateral sclerosis; a gene involved in pathogenesis of Parkinson's disease and/or Alzheimer's disease; apolipoprotein B (APOB) and proprotein convertase subtilisin/kexin type 9 (PCSK9), hypercholesterolemia; HIV Tat, human immunodeficiency virus transactivator of transcription gene, in HIV infection; HIV TAR, HIV TAR, human immunodeficiency virus transactivator response element gene, in HIV infection; C-C chemokine receptor (CCR5) in HIV infection; Rous sarcoma virus (RSV) nucleocapsid protein in RSV infection, liver-specific microRNA (miR-122) in hepatitis C virus infection; p53, acute kidney injury or delayed graft function kidney transplant or kidney injury acute renal failure; protein kinase N3 (PKN3) in advance recurrent or metastatic solid malignancies; LMP2, LMP2 also known as proteasome subunit beta-type 9 (PSMB 9), metastatic melanoma; LMP7,also known as proteasome subunit beta-type 8 (PSMB 8), metastatic melanoma; MECL1 also known as proteasome subunit beta-type 10 (PSMB 10), metastatic melanoma; vascular endothelial growth factor (VEGF) in solid tumors; kinesin spindle protein in solid tumors, apoptosis suppressor B- cell CLL/lymphoma (BCL-2) in chronic myeloid leukemia; ribonucleotide reductase M2 (RRM2) in solid tumors; Furin in solid tumors; polo-like kinase 1 (PLK1) in liver tumors, diacylglycerol acyltransferase 1 (DGAT1) in hepatitis C infection, beta-catenin in familial adenomatous polyposis; beta2 adrenergic receptor, glaucoma; RTP801/Reddl also known as DNA damage-inducible transcript 4 protein, in diabetic macular edema (DME) or age- related macular degeneration; vascular endothelial growth factor receptor I (VEGFR1) in age-related macular degeneration or choroidal neovascularization, caspase 2 in non-arteritic ischaemic optic neuropathy; Keratin 6 A N 17K mutant protein in pachyonychia congenital; influenza A virus genome/gene sequences in influenza infection; severe acute respiratory syndrome (SARS) coronavirus genome/gene sequences in SARS infection; respiratory syncytial virus genome/gene sequences in respiratory syncytial virus infection; Ebola filovirus genome/gene sequence in Ebola infection; hepatitis B and C virus genome/gene sequences in hepatitis B and C infection; herpes simplex virus (HSV) genome/gene sequences in HSV infection, coxsackievirus B3 genome/gene sequences in coxsackievirus B3 infection; silencing of a pathogenic allele of a gene (allele-specific silencing) like torsin A (TORI A) in primary dystonia, pan-class I and HLA-allele specific in transplant; and mutant rhodopsin gene (RHO) in autosomal dominantly inherited retinitis pigmentosa (adRP); and wherein said inhibitory nucleic acid is optionally a siRNA, an antisense molecule, miRNA, RNAi, a ribozyme or a shRNA.
